(19) Europäisches Patentamt
European Patent Office
Office européen des brevets

(11) EP 2 015 069 A1

(12) # EUROPEAN PATENT APPLICATION
published in accordance with Art. 153(4) EPC

(43) Date of publication:
**14.01.2009 Bulletin 2009/03**

(21) Application number: **07742318.4**

(22) Date of filing: **24.04.2007**

(51) Int Cl.:
***G01N 33/493*** *(2006.01)*

(86) International application number:
**PCT/JP2007/058883**

(87) International publication number:
**WO 2007/123245 (01.11.2007 Gazette 2007/44)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **24.04.2006 JP 2006119559**

(71) Applicant: **Panasonic Corporation**
**Kadoma-shi**
**Osaka 571-8501 (JP)**

(72) Inventors:
- **KENJOU, Noriko,**
  **c/o Panasonic Corporation, IPROC, IP Dev. Center**
  **Chuo-ku, Osaka-shi, Osaka 540-6207 (JP)**
- **TANIIKE, Yuko,**
  **c/o Panasonic Corp., IPROC, IP Developm. Cent.**
  **Chuo-ku, Osaka-shi, Osaka 540-6207 (JP)**
- **KAWAMURA, Tatsurou**
  **c/o Panasonic Corp., IPROC, IP Developm. Cent.**
  **Chuo-ku, Osaka-shi, Osaka 540-6207 (JP)**
- **SHIMBA, Noriko**
  **c/ Panasonic Corp., IPROC IP, Developm. Cent.**
  **Chuo-ku, Osaka-shi, Osaka 540-6207 (JP)**
- **UESHIMA, Hirotsugu**
  **c/o Shiga University of Medical Science**
  **Shiga 520-2192 (JP)**
- **OKAMURA, Tomonori**
  **c/o Shiga University of Medical Science,**
  **Shiga 520-2192 (JP)**
- **HOZAWA, Atsushi**
  **c/o Shiga University of Medical Science,**
  **Shiga 520-2192 (JP)**

(74) Representative: **Pautex Schneider, Nicole Véronique et al**
**Novagraaf International SA**
**25, avenue du Pailly**
**1220 Les Avanchets - Geneva (CH)**

(54) **METHOD OF MEASURING DAILY URINARY EXCRETION AND APPARATUS FOR MEASURING DAILY URINARY EXCRETION**

(57) A daily urinary excretion measurement apparatus and a method thereof, for measuring daily excretion of a urine component highly accurately and easily, are provided.

A daily urinary excretion measurement apparatus (100) includes: a urine component calculation unit (411) which measures the amount of the urine component included in before-bedtime urine that is excreted for the last time before bedtime in the day and the amount (salt) of the urine component (salt) included in overnight urine that is excreted for the first time after wake-up on the next day following a sleep; a regression equation storage unit (416) which stores a correlation (a regression equation) between the amount of the before-bedtime urine component and the amount of the overnight urine component and a total amount of the urine component included in daily urine; a daily urinary excretion calculation unit (413) which calculates the total amount of the urine component excreted in the daily urine according to the regression equation stored in the regression equation storage unit (416), using the amount of the before-bedtime urine component (salt) and the amount of the overnight urine component (salt) calculated by the urine component calculation unit (411).



Printed by Jouve, 75001 PARIS (FR)

(Cont. next page)

FIG. 3

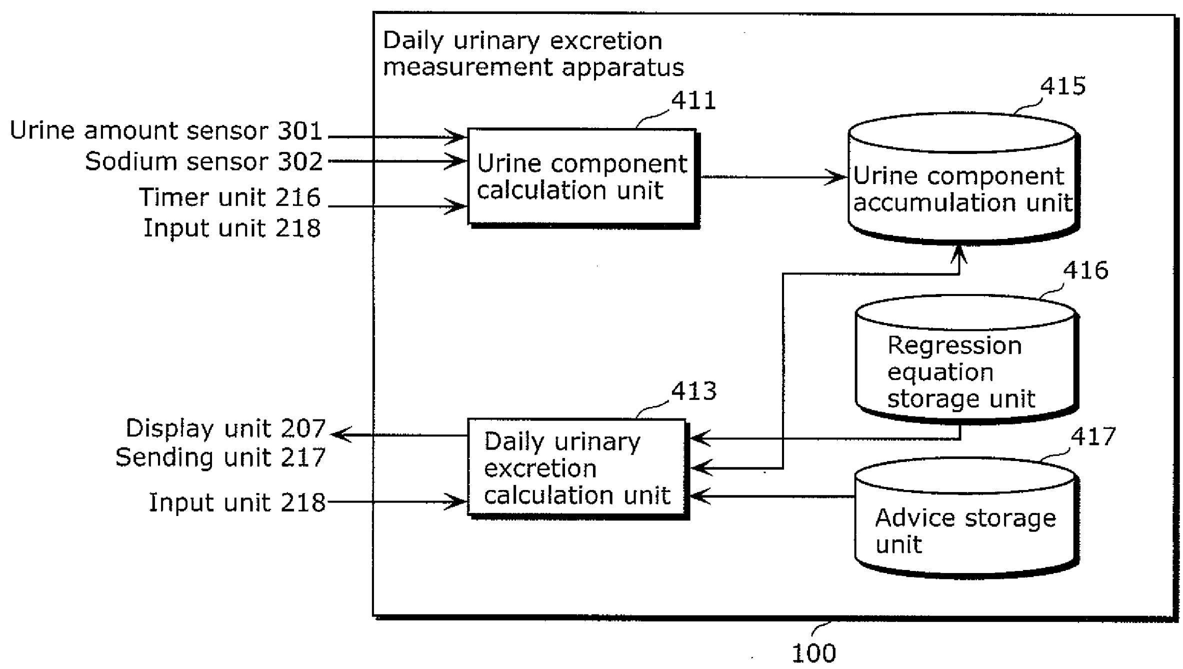
Daily urinary excretion measurement apparatus
411
415
Urine amount sensor 301
Sodium sensor 302
Timer unit 216
Input unit 218
Urine component calculation unit
Urine component accumulation unit
416
Regression equation storage unit
413
Display unit 207
Sending unit 217
Input unit 218
Daily urinary excretion calculation unit
417
Advice storage unit
100

## Description

### Technical Field

The present invention relates to an apparatus and a method for measuring an excreted amount of a urine component so that a total amount of the urine component excreted per day is estimated.

### Background Art

Urine tests have been widely used over a number of years since it is easy to take urine samples, an examination can be carried out noninvasively, and no invasion such as pain is accompanied during urine collection. Urine includes various kinds of components, such as salt, sugar, and protein, each concentration of which varies depending on a living condition and a health state of a subject. Thus, easy measurements of such components at home are useful for healthcare. For example, excessive salt intake can cause hypertension and stomach cancer. Although the amount of intake should be managed, it is complicated and burdensome to measure the amount of salt for each food to be eaten and to calculate the total amount. With this being the situation, a method of measuring the urinary sodium excretion so as to manage the daily salt intake has been suggested (see Patent Reference 1, for example). FIG. 1 is a diagram showing a conventional salt intake measurement apparatus described in Patent Reference 1.

Using the salt intake measurement apparatus shown in FIG. 1, from a sodium concentration of first early-morning urine that is measured by a sensor unit 41 (this urine is excreted for the first time after wake-up in the morning, and is referred to as "overnight urine" hereafter) and the overnight urine received by an input unit 42, the amount of sodium in the overnight urine of a user is calculated by a calculation unit. Moreover, daily urinary sodium excretion is calculated according to a regression equation previously stored in the salt intake measurement apparatus, and is digitally displayed on a display unit 43.

In the case of the configuration shown in FIG. 1, the amount of sodium in the overnight urine is measured by multiplying the sodium concentration of the overnight urine by the amount of the overnight urine. Then, the daily urinary sodium excretion is calculated according to a correlation between the sodium excretion in the overnight urine and the daily urinary sodium excretion (see Patent Reference 1).

Also, a method of estimating the daily urinary excretion from the overnight urine corrected on the basis of time (a period of time elapsed after the previous urination is converted into eight hours to correct the amount of urine) is under consideration (see Non-patent Reference 1).

Patent Reference 1: Japanese Unexamined Patent Application Publication No. 2002-267662
Non-patent Reference 1: Kotaro Yamasue et al. "Methods for self-monitoring of daily salt and potassium intake at home" by JJCDP, Vol. 39, No. 3, pp.157-163, 2004.

### Disclosure of Invention

### Problems that Invention is to Solve

However, although it is possible for most people to collect their overnight urine, there is a report that the overnight urine has a low correlation with the accumulated urine excreted within 24 hours (referred to as "24-hour urine" hereafter). It is considered that the correlation between the overnight urine and the 24-hour urine is low because: (1) the volume of meals during nighttime hours and the mealtime are different for each individual; (2) the amount of overnight urine is different for each individual due to individual differences in antidiuretic hormone (ADH) secreted by pituitary gland in the brain during the night; and (3) renal function is different for each individual. In addition, another reason is (4) that the amount of salt excretion in the overnight urine is changeable depending on the amount of exercise loaded during the day. For these reasons, when the daily excretion is estimated from the overnight urine, there is a disadvantage that it is difficult to obtain a value which is precise and excellent in reproducibility unless an appropriate correction is performed.

Moreover, using the method of estimating the daily urinary excretion from the overnight urine corrected on the basis of a period of time (the amount of urine is converted into the amount to be excreted in eight hours) as described in Non-patent Reference 1, there may be a case where the correction is not appropriate.

The present invention is to solve the stated conventional problems and has an object of providing a daily urinary excretion measurement method whereby daily excretion of a urine component can be measured highly accurately and easily, and of providing a measurement apparatus employing this method.

## Means to Solve the Problems

To solve the stated conventional problems, the present invention provides a daily urinary excretion measurement method for measuring a total amount of a urine component excreted in daily urine, the method including: collecting a plurality of spot urine samples (each of which is collected per urination) out of spot urine samples excreted within a predetermined period of time, and calculating an amount of the urine component for each spot urine sample using the collected plurality of spot urine samples, the collecting and the calculating being performed by a urine component calculation unit; and holding a correlation between the amounts of the urine component included in the plurality of spot urine samples excreted within the predetermined period of time and a daily urinary excretion which is the total amount of the urine component included in the daily urine, and calculating the daily urinary excretion on the basis of the correlation and the amounts of the urine component included in the plurality of spot urine samples calculated by the urine component calculation unit, the holding and the calculating of the daily urinary excretion being performed by a daily urinary excretion calculation unit.

Moreover, the present invention provides a daily urinary excretion measurement apparatus which measures a total amount of a urine component excreted in daily urine, the apparatus including: a urine component calculation unit which collects a plurality of spot urine samples (each of which is collected per urination) out of spot urine samples excreted within a predetermined period of time, and calculates an amount of a urine component for each spot urine sample using the collected plurality of spot urine samples; and a daily urinary excretion calculation unit which holds a correlation between the amounts of the urine component included in the plurality of spot urine samples excreted within the predetermined period of time and a daily urinary excretion which is the total amount of the urine component included in the daily urine, and calculates the daily urinary excretion on the basis of the correlation and the amounts of the urine component included in the plurality of spot urine samples calculated by the urine component calculation unit.

It should be noted that the present invention can be realized not only as an apparatus, but also as: a method having the processing units included in the apparatus as its steps; a program causing a computer to execute these steps; a computer-readable recording medium, such as a CD-ROM, which records the program; and; information, data, or a signal showing the program. The program, the information, the data, or the signal may be distributed via a communication network such as the Internet.

## Effects of the Invention

Using the daily urinary excretion measurement method and the daily urinary excretion measurement apparatus of the present invention, a daily excretion of a urine component can be measured highly accurately and easily through a small effort (such as the number of measurements).

## Brief Description of Drawings

FIG. 1 is a diagram showing a conventional salt intake measurement apparatus.

FIG. 2 is a diagram showing a hardware configuration of a daily urinary excretion measurement apparatus according to a first embodiment of the present invention.

FIG. 3 is a function block diagram showing major units included in the daily urinary excretion measurement apparatus.

FIG. 4 is a diagram showing an external view of the daily urinary excretion measurement apparatus and showing a method of measuring a urine component using the daily urinary excretion measurement apparatus.

FIG. 5 is a diagram of a perspective view showing an appearance of a control panel part of a control unit included in the daily urinary excretion measurement apparatus.

FIG. 6 is a flowchart showing a measurement control routine executed by a CPU of the daily urinary excretion measurement apparatus.

FIG. 7 is a graph showing an example of a regression equation stored in a regression equation storage unit of the daily urinary excretion measurement apparatus.

FIG. 8 is a block diagram showing a configuration of a daily urinary excretion measurement apparatus which calculates daily urinary excretion through performing 8-hour correction on overnight urinary sodium excretion, according to another embodiment of the present invention.

FIG. 9 is a diagram of an external view showing an appearance of a control panel of the daily urinary excretion measurement apparatus.

FIG. 10 is a block diagram showing a configuration of a daily urinary excretion measurement apparatus which formulates a regression equation for each user, according to another embodiment of the present invention.

FIG. 11 is a flowchart showing a flow of processing performed when the daily urinary excretion measurement apparatus formulates a regression equation for each user.

FIG. 12 is a diagram showing a hardware configuration of a daily urinary excretion measurement apparatus according to a fourth embodiment.

FIG. 13 is a function block diagram showing major units included in the daily urinary excretion measurement apparatus realized by a CPU and a memory shown in FIG. 12.

FIG. 14 is a graph showing the number of period-difference detections in the case of using an average value of data obtained through a plurality of measurements that include both the overnight urine measurement and the before bedtime urine measurement.

FIG. 15 is a graph showing the number of period-difference detections in the case of using an average value of data obtained through a plurality of measurements that include only the overnight urine measurements.

FIG. 16 is a graph showing the number of period-difference detections in the case of using an average value of data obtained through a plurality of measurements that include only the before bedtime urine measurements.

**Numerical References**

| | |
|---|---|
| 1 | beaker |
| 2 | control unit |
| 3 | sensor |
| 4 | urine |
| 41 | sensor unit |
| 42 | input unit |
| 43 | display unit |
| 100, 200, 300, 400 | daily urinary excretion measurement apparatuses |
| 201 | connection unit |
| 202 | cursor leftward-move button |
| 203 | cursor rightward-move button |
| 204 | measurement start button |
| 205 | excretion time information button |
| 206 | measurement result sending button |
| 207 | display unit |
| 211 | CPU |
| 215 | memory |
| 216 | timer unit |
| 217 | sending unit |
| 218 | input unit |
| 230 | excretion time information buttons |
| 240 | result display button |
| 250 | cursor move buttons |
| 260 | measurement notification menu button |
| 301 | urine amount sensor |
| 302 | sodium sensor |
| 303 | creatinine sensor |

| | |
|---|---|
| 411, 911 | urine component calculation units |
| 413, 713 | daily urinary excretion calculation units |
| 415, 715 | urine component accumulation units |
| 416, 716 | regression equation storage units |
| 417 | advice storage unit |
| 418 | average daily urinary excretion calculation unit |
| 712 | 8-hour correction unit |
| 714 | excretion time information judgment unit |
| 717 | notification menu display unit |
| 718 | notification time table storage unit |
| 914 | regression equation calculation unit |

**Best Mode for Carrying Out the Invention**

The present invention provides a daily urinary excretion measurement method for measuring a total amount of a urine component excreted in daily urine, the method including: collecting a plurality of spot urine samples (each of which is collected per urination) out of spot urine samples excreted within a predetermined period of time, and calculating an amount of the urine component for each spot urine sample using the collected plurality of spot urine samples, the collecting and the calculating being performed by a urine component calculation unit; and holding a correlation between the amounts of the urine component included in the plurality of spot urine samples excreted within the predetermined period of time and a daily urinary excretion which is the total amount of the urine component included in the daily urine, and calculating the daily urinary excretion on the basis of the correlation and the amounts of the urine component included in the plurality of spot urine samples calculated by the urine component calculation unit, the holding and the calculating of the daily urinary excretion being performed by a daily urinary excretion calculation unit.

Moreover, the present invention provides a daily urinary excretion measurement apparatus which measures a total amount of a urine component excreted in daily urine, the apparatus including: a urine component calculation unit which collects a plurality of spot urine samples (each of which is collected per urination) out of spot urine samples excreted within a predetermined period of time, and calculates an amount of a urine component for each spot urine sample using the collected plurality of spot urine samples; and a daily urinary excretion calculation unit which holds a correlation between the amounts of the urine component included in the plurality of spot urine samples excreted within the predetermined period of time and a daily urinary excretion which is the total amount of the urine component included in the daily urine, and calculates the daily urinary excretion on the basis of the correlation and the amounts of the urine component included in the plurality of spot urine samples calculated by the urine component calculation unit.

Biological rhythms of urine component excretion are different between a time of day for activities including having meals and exercising and a time of day for sleeping. For this reason, when the amount of daily urine component excretion is estimated, it is necessary to select a urine sample with consideration given to the biological rhythms. The urine sample is selected as appropriate, depending on a subject of the measurement. Still, in order to give consideration to the biological rhythms, the last urine of the day before bedtime may be used as a urine sample of the time of day for activities whereas the overnight urine may be used as a sample of the time of day for sleeping. In addition, the last urine before bedtime and the overnight urine are easy to sample for most people because it is a time of day for them to be home. Thus, this daily urinary excretion measurement method can be used more widely and commonly. Accordingly, using the daily urinary excretion measurement method and the daily urinary excretion measurement apparatus of the present invention, the daily excretion of a urine component can be measured highly accurately and easily since consideration is given to the biological rhythms.

Also, according to the daily urinary excretion measurement method of the present invention, it is preferable that, in the calculating of the daily urinary excretion, the daily urinary excretion calculation unit calculates the daily urinary excretion of the urine component using the amounts of the urine component included in two of the spot urine samples collected within the predetermined period of time.

According to the daily urinary excretion measurement method of the present invention, it is preferable that, in the calculating of the amount of the urine component, the urine component calculation unit calculates the amount of the urine component for each of the plurality of spot urine samples using the plurality of spot urine samples excreted within twenty-four hours.

According to the daily urinary excretion measurement method of the present invention, it is preferable that, in the calculating of the amount of the urine component, the urine component calculation unit calculates the amount of the urine component for each of the plurality of spot urine samples using a component concentration of the spot urine sample measured by a component sensor and a urine amount measured by a urine amount sensor.

According to the daily urinary excretion measurement method of the present invention, it is preferable that, in the calculating of the amount of the urine component, the urine component calculation unit calculates the amount of the urine component for each of the plurality of spot urine samples of before-bedtime urine and overnight urine, the before-bedtime urine being excreted and collected for the last time before bedtime and the overnight urine being excreted and collected for the first time after wake-up following a sleep, and that, in the calculating of the daily urinary excretion, the daily urinary excretion calculation unit calculates the daily urinary excretion of the urine component using the amount of the before-bedtime urine component calculated by the urine component calculation unit and the amount of the overnight urine component calculated by the urine component calculation unit, on the basis of a correlation between: the amount of the before-bedtime urine component and the amount of the overnight urine component; and the total amount of the urine component included in the daily urine. Accordingly, since the urine samples which are produced at a time of day for activities and at a time of day for sleeping are used, the daily excretion of the urine component can be measured with an extremely high degree of accuracy.

According to the daily urinary excretion measurement method of the present invention, it is preferable that the method further includes: receiving an input of excretion time information regarding an excretion time of the spot urine sample, the receiving being performed by an input unit; accumulating the amount of the urine component calculated by the urine component calculation unit for each of the spot urine samples in association with the excretion time information of the spot urine sample received by the input unit; and performing an 8-hour correction on the amount of the overnight urine component by converting a period of time taken from an excretion time of the before-bedtime urine to an excretion time of the overnight urine into 8 hours, on the basis of the pieces of the excretion time information accumulated respectively in association with the amount of before-bedtime urine component and the amount of the overnight urine component, wherein, in the calculating of the daily urinary excretion, the daily urinary excretion calculation unit calculates the daily urinary excretion of the urine component using: the amount of the before-bedtime urine component calculated by the urine component calculation unit; and the amount of the overnight urine component calculated by the urine component calculation unit and corrected by the daily urinary excretion calculation unit in the performing of the 8-hour correction, on the basis of a correlation between: a sum of the before-bedtime urine component amount and the 8-hour corrected overnight urine component amount; and the total amount of the urine component included in the daily urine. In this way, by performing the 8-hour correction on the overnight urine, the daily excretion of the urine component can be measured with a higher degree of accuracy.

Moreover, according to the daily urinary excretion measurement method of the present invention, it is preferable that, in the calculating of the amount of the urine component, the urine component calculation unit calculates a ratio of the urine component to creatinine in the spot urine sample as the amount of the urine component included in the spot urine sample, from a component concentration of the spot urine sample measured by a component sensor and a creatinine concentration of the spot urine sample measured by a creatinine sensor, and that, in the calculating of the daily urinary excretion, the daily urinary excretion calculation unit calculates the daily urinary excretion of the urine component, using: the creatinine ratio which is calculated as the amount of the urine component by the urine component calculation unit; and an amount of daily creatinine excretion held in advance by the urine component calculation unit. With this, the user does not need to collect all the spot urine samples, so that the burden of the measurement of the user is reduced. On account of this, a simple and easy measurement becomes possible.

According to the daily urinary excretion measurement method of the present invention, it is preferable that, in the calculating of the amount of the daily urinary excretion, the daily urinary excretion calculation unit calculates the daily urinary excretion using the amounts of the urine component of at least three but not exceeding fourteen spot urine samples calculated by the urine component calculation unit within the predetermined period of time. In this way, by using data obtained through three or more times of measurements, the average daily excretion of the urine component within a certain period of time can be grasped with a high degree of accuracy.

According to the daily urinary excretion measurement method of the present invention, it is preferable that, in the calculating of the amount of the urine component, the urine component calculation unit calculates the amounts of the urine component of at least three but not exceeding fourteen spot urine samples collected out of the spot urine samples excreted in seven days. When the stated period of time exceeds seven days, a lifestyle habit may change significantly. For this reason, by setting the evaluation time within seven days, it becomes possible to obtain a result reflecting the lifestyle habit of the user.

According to the daily urinary excretion measurement method of the present invention, it is preferable that, in the calculating of the amount of the urine component, the urine component calculation unit calculates the amounts of the urine component of the spot urine samples including at least two pairs of the before-bedtime urine sample and the overnight urine sample out of the collected spot urine samples, each of the pairs sandwiching a same sleep. As described so far, according to the daily urinary excretion measurement method of the present invention, since the daily excretion of the urine component is measured using the urine samples which are produced at a time of day for activities and at a time of day for sleeping, consideration is given to both of the biological rhythms. Thus, a measurement result of the daily urinary excretion can be obtained with a high degree of accuracy.

Also, according to the daily urinary excretion measurement method of the present invention, it is preferable that, in the calculating of the amount of the urine component, the urine component calculation unit calculates the amounts of the urine component of the collected spot urine samples, all of which are the overnight urine samples. The overnight urine, which is the first morning urine after wake-up, is easy to collect and, therefore, the measurement can be easily continued.

Moreover, according to the daily urinary excretion measurement method of the present invention, it is preferable that, in the calculating of the amount of the urine component, the urine component calculation unit calculates the amounts of the urine component of the collected spot urine samples, all of which are the before-bedtime urine samples. When the user is a worker or a full-time homemaker, early morning hours when the overnight urine is to be collected are busy time. Thus, a collection error may take place. Also, provided that the measurement is carried out at home, it is easier to collect the before-bedtime urine.

Furthermore, according to the daily urinary excretion measurement apparatus of the present invention, it is preferable that the urine component calculation unit includes: a component sensor which measures a component concentration of the spot urine sample; and a urine amount sensor which measures a urine amount of the spot urine sample, and that the urine component calculation unit calculates the amount of the urine component included in the spot urine sample from the component concentration measured by the component sensor and the urine amount measured by the urine amount sensor.

According to the daily urinary excretion measurement apparatus of the present invention, it is preferable that the urine component calculation unit includes: a component sensor which measures a component concentration of the spot urine sample; and a creatinine sensor which measures a creatinine concentration of the spot urine sample, and that the urine component calculation unit calculates a ratio of the urine component to creatinine in the spot urine sample as the amount of the urine component included in the spot urine sample, from the component concentration measured by the component sensor and the creatinine concentration measured by the creatinine sensor.

It is preferable that the daily urinary excretion measurement apparatus of the present invention further includes a display unit which displays one of: each amount of the urine component in the plurality of spot urine samples calculated by the urine component calculation unit; and the daily urinary excretion calculated by the daily urinary excretion calculation unit.

Also, according to the daily urinary excretion measurement apparatus of the present invention, it is preferable that the urine component calculation unit calculates the amount of the urine component for each of the plurality of spot urine samples of before-bedtime urine and overnight urine, the before-bedtime urine being excreted and collected for the last time before bedtime and the overnight urine being excreted and collected for the first time after wake-up following a sleep, and that the daily urinary excretion measurement apparatus further includes a display unit which displays one of: the amount of the before-bedtime urine component calculated by the urine component calculation unit; the amount of the overnight urine component calculated by the urine component calculation unit; and the daily urinary excretion calculated by the daily urinary excretion calculation unit. With this configuration, the measurement data and the analysis data can be displayed and the daily excretion of the user can be grasped. Also, the daily urinary excretion measurement apparatus having an increased functionality can be provided using: a data input screen; an output screen to display the measurement data and the analysis data; and an instruction screen to display a measurement instruction.

Moreover, it is preferable that the daily urinary excretion measurement apparatus of the present invention further includes a display unit which displays the daily urinary excretion calculated by the daily urinary excretion calculation unit only after the urine component calculation unit calculates the amount of the urine component for each of the plurality of spot urine samples collected within the predetermined period of time. With this configuration, the measurement result is not shown before the specified number of measurements are ended. Accordingly, it becomes possible to present a more accurate result to the user.

Furthermore, it is preferable that the daily urinary excretion measurement apparatus of the present invention further includes: a standard amount reception unit which receives an input of a standard amount of the total amount of the urine component excreted in the daily urine; a comparison unit which compares the daily urinary excretion calculated by the daily urinary excretion calculation unit and the standard amount received by the standard amount reception unit, and judges whether or not the calculated daily urinary excretion exceeds the standard amount; and a display unit which displays an advice when the comparison unit judges that the daily urinary excretion exceeds the standard amount. With this configuration, the user can grasp the user's own daily urinary excretion. Also, the user can be aware of how different the user's own daily urinary excretion is from a target value or a standard value such as a recommended value, and this is useful for the self care. Moreover, health advices and messages become an encouragement of the self care and a gathering of knowledge about proper healthcare.

It is preferable that the daily urinary excretion measurement apparatus of the present invention further includes a measurement notification unit which notifies a user of a timing at which the amount of the urine component is to be calculated by the urine component calculation unit. With this configuration, by setting a urination time, as the stated timing, having a high correlation with the daily urinary sodium, it becomes easy to perform the measurement at the time represented by the excretion time information having a high correlation with the daily urinary sodium. This leads to a more accurate estimation of the daily excretion.

Also, it is preferable that the daily urinary excretion measurement apparatus of the present invention further includes: a urine component accumulation unit which accumulates the amounts of the before-bedtime urine component and the amounts of the overnight urine component of at least three days together with the respective corresponding daily urinary excretions; and a correlation calculation unit which calculates, from: a sum of the amount of the before-bedtime urine component and the amount of the overnight urine component; and the corresponding daily urinary excretion accumulated in the urine component accumulation unit, a correlation between: the sum of the amount of the before-bedtime urine component and the amount of the overnight urine component; and the corresponding daily urinary excretion according to a linear approximation. With this, the daily urinary excretion measurement apparatus can obtain the regression equation for each individual user, that expresses the correlation between: the sum of the before-bedtime urine component excretion and the overnight urine component excretion; and the total daily amount of the urine component excretion. Accordingly, a more accurate daily excretion can be calculated.

The following is a detailed description of embodiments of the present invention with reference to the drawings.

(First embodiment)

<before-bedtime urinary sodium excretion + overnight urinary sodium excretion>

FIG. 2 is a diagram showing a hardware configuration of a daily urinary excretion measurement apparatus according to the present embodiment. The daily urinary excretion measurement apparatus 100 measures the amount of urine per urination and the amount of a urine component included in the urine. Using these, the daily urinary excretion measurement apparatus 100 measures the amount of before-bedtime urine component excretion which is the amount of the urine component included in the urine excreted the last time before bedtime and the amount of overnight urine component excretion which is the amount of the urine component included in the urine excreted for the first time in the morning after wake-up (this urine is referred to as the "overnight urine" hereafter). By these measurements, the daily urinary excretion measurement apparatus 100 automatically calculates the total amount of the urine component excreted in the urine of the whole day (for example, the past day starting from the time of the first urination after wake-up). The daily urinary excretion measurement apparatus 100 includes a control unit 2 and a sensor 3. The control unit 2 includes a display unit 207, a CPU 211, a memory 215, a timer unit 216, a sending unit 217, and an input unit 218, which are interconnected via buses. The sensor 3 includes a urine amount sensor 301 and a sodium sensor 302.

The display unit 207 includes a liquid crystal display. However, it is not especially limited to this as long as it displays measurement data and analysis data. The display unit 207 displays: a data input screen; an output screen for displaying the measurement data and the analysis data; and an instruction screen for displaying a measurement instruction. To be more specific, the display unit 207 corresponds to "a display unit which displays one of: the amount of the before-bedtime urine component calculated by the urine component calculation unit; the amount of the overnight urine component calculated by the urine component calculation unit; and the daily urinary excretion calculated by the daily urinary excretion calculation unit" and displays a measured amount of urine, a concentration of a urine component, an amount of a urine component, and a calculated amount of daily urinary excretion. In addition, the display unit 207 corresponds to "a display unit which displays an advice when the comparison unit judges that the daily urinary excretion exceeds the standard amount", and displays a health advice on the basis of the calculated daily urinary excretion. Moreover, the display unit 207 displays a message screen for notifying the user of a preferable timing for measurement. The CPU 211 is responsible for controlling the entire control unit 2. In accordance with a program stored in a ROM, the CPU 211 performs secondary processing on the measurement data, such as processing of urine measurements using the sensors, processing of calculating the amount of urinary sodium by multiplying the measured urinary sodium concentration by the amount of urine; and processing of estimating the amount of daily urinary excretion from the amount of urinary sodium excretion. Moreover, the CPU 211 receives an instruction from the user and displays corresponding information on the display unit 207 by exchanging signals with the input unit 218 and a touch panel included in a control panel.

The memory 215 includes a RAM, a ROM, an IC memory, and a hard disk. For example, the memory 215 previously stores, in the ROM r the like, a correlation between: a sum of the amount of the urine component included in the before-bedtime urine and the amount of the urine component included in the overnight urine; and the amount of daily urinary excretion. Also, the readable/writable large-capacity memory 215, which is realized by the hard disk, the IC memory, and the like, accumulates the measurement data, such as the measured amount of urine, the concentration of the urine component, and the amount of the urine component, in association with excretion time information for each measurement.

The timer unit 216 is a timer or the like, and detects urination time information.

The sending unit 217 sends a measurement result, a health advice, or the like to an external personal computer, a cellular telephone, a PDA, or the like which is registered in advance by the user.

The input unit 218 includes a various kinds of buttons provided on the control unit 2 and a touch panel provided on the display unit 207.

It should be noted that, although illustration is omitted, the daily urinary excretion measurement apparatus 100 of the present embodiment includes an input/output port and so can perform input and output of data into and from a flexible disk and the IC memory via a flexible disk drive or an IC memory drive externally connected to the input/output port.

The urine amount sensor 301 measures the amount of urine sucked into the sensor 3. To be more specific, the amount of urine is measured according to a resistance measurement method. On the basis that a resistance of a resistor included in the sensor 3 which is immersed in a urine collection container (a beaker 1) varies depending on the amount of urine, a calibration curve representing a relation between the resistance and the amount of urine is formed so that the amount of urine is calculated from a resistance value. More specifically, on the basis that the resistance of the resistor immersed to the bottom of the container (the beaker 1) storing liquid varies depending on the amount of liquid, a calibration curve representing a relation between the resistance and the amount of liquid is formed so that the amount of urine is calculated from a resistance value. A calibration curve representing a relation between inverted voltage and liquid amount is obtained using saline solution, which is then applied to urine. The amount of urine obtained though this method is almost the same value that is obtained through a weight measurement method using a balance.

The sodium sensor 302 measures a urinary sodium concentration of the urine sucked into the sensor 3. The sodium sensor 302 measures the urinary sodium concentration according to an electrode method employing an electrical conductivity method. The measurement of the electrical conductivity can be realized by measuring impedance between predetermined terminals. Since having no moving parts, the configuration is simple and thus its operation reliability is high. The electrical conductivity of urine generally depends on the sodium concentration. On account of this, a calibration curve may be previously obtained from a correlation between the urinary sodium concentration and the electrical conductivity, and then the sodium concentration can be measured using an inexpensive sensor which has two electrodes for measuring the electrical conductivity. It should be noted that when the sodium concentration is measured on the basis of the electrical conductivity, correction may be performed in order to remove an error caused by potassium, magnesium, and the like other than sodium.

FIG. 3 is a function block diagram showing major units included in the daily urinary excretion measurement apparatus 100 which is realized by the CPU 211 and the memory 215 shown in FIG. 2. As shown in this diagram, the daily urinary excretion measurement apparatus 100 includes a urine component calculation unit 411, a daily urinary excretion calculation unit 413, a urine component accumulation unit 415, a regression equation storage unit 416, and an advice storage unit 417.

The daily urinary excretion measurement 100 corresponds to "a daily urinary excretion measurement apparatus which measures a total amount of a urine component excreted in daily urine, the apparatus including: a urine component calculation unit which collects a plurality of spot urine samples (each of which is collected per urination) out of spot urine samples excreted within a predetermined period of time, and calculates an amount of a urine component for each spot urine sample using the collected plurality of spot urine samples; and a daily urinary excretion calculation unit which holds a correlation between the amounts of the urine component included in the plurality of spot urine samples excreted within the predetermined period of time and a daily urinary excretion which is the total amount of the urine component included in the daily urine, and calculates the daily urinary excretion on the basis of the correlation and the amounts of the urine component included in the plurality of spot urine samples calculated by the urine component calculation unit." In this case, the urine component calculation unit 411 corresponds to the "urine component calculation unit"; the regression equation corresponds to the "correlation"; and the regression equation storage unit 416 and the daily urinary excretion calculation unit 413 correspond to the "daily urinary excretion calculation unit".

Moreover, the daily urinary excretion measurement apparatus 100 corresponds to "the daily urinary excretion measurement apparatus, further including: a standard amount reception unit which receives an input of a standard amount of the total amount of the urine component excreted in the daily urine; a comparison unit which compares the daily urinary excretion calculated by the daily urinary excretion calculation unit and the standard amount received by the standard amount reception unit, and judges whether or not the calculated daily urinary excretion exceeds the standard amount; and a display unit which displays an advice when the comparison unit judges that the daily urinary excretion exceeds the standard amount." The input unit 218 corresponds to the "standard amount reception unit"; the daily urinary excretion calculation unit 413 corresponds to the "comparison unit"; and the advice storage unit 417 and the display unit 207 corresponds to the "display unit".

The urine component calculation unit 411 calculates the amount of urinary sodium included in the urine from the amount of urine received from the urine amount sensor 301 and the sodium concentration received from the sodium sensor 302. Moreover, the urine component calculation unit 411 accumulates the received urine amount, the received sodium concentration, and the calculated urinary sodium amount into the urine component accumulation unit 415 in association with the current-time of measurement obtained from the timer unit 216 and the excretion time information received from the input unit 218.

When the overnight urinary sodium amount is calculated by the urine component calculation unit 411, the daily urinary excretion calculation unit 413 reads the before-bedtime urinary sodium amount which is the amount of sodium included in the before-bedtime urine and the overnight urinary sodium amount which is the amount of sodium included in the overnight urine, from the urine component accumulation unit 415. Following this, the daily urinary excretion calculation unit 413 substitutes a value obtained by summing the read before-bedtime urinary sodium amount and the read overnight urinary sodium amount into the regression equation stored in the regression equation storage unit 416, so that the daily urinary sodium excreted in the urine of the user is calculated. Also, the daily urinary excretion calculation unit 413 reads an advice corresponding to the calculated daily urinary excretion from the advice storage unit 417 and causes the display unit 207 to display the advice. Moreover, following the instruction received from the input unit 218, the daily urinary excretion calculation unit 413 sends information including the calculated daily urinary excretion and the corresponding advice, to the sending unit 217.

For each urination, the urine component accumulation unit 415 accumulates the current time of measurement, the excretion time information, the amount of urine, the sodium concentration, the urinary sodium amount, the daily urinary excretion, the daily urinary sodium excretion, and a difference from a standard amount. The regression equation storage unit 416 stores a regression equation, which is previously calculated, that represents a correlation between: a sum of the before-bedtime urinary sodium amount and the overnight urinary sodium amount; and the daily urinary sodium excretion amount. The advice storage unit 417 stores a predetermined health advice corresponding to a standard amount of the daily urinary sodium excretion or a predetermined health advice corresponding to the difference from the standard amount.

FIG. 4 is a diagram showing an external view of the daily urinary excretion measurement apparatus 100 shown in FIG. 2 and showing a method of measuring a urine component using the daily urinary excretion measurement apparatus. FIG. 5 is a diagram of a perspective view showing an appearance of a control panel part of the control unit 2 included in the daily urinary excretion measurement apparatus 100 shown in FIG. 4.

(Appearance of daily urinary excretion measurement apparatus)

As shown in FIGS. 4 and 5, the sensor 3 is removable from the control unit 2 via a connection unit 201, so can be replaced when exhausted. A protrusion of the sensor 3, which is not shown in the diagram, can be fit into the connection unit 201. A terminal for electrically connecting the sensor 3 and the control unit 2 is placed inside the connection unit 201. On its control panel, the control unit 2 includes a cursor leftward-move button 202, a cursor rightward-move button 203, a measurement start button 204, an excretion time information button 205, a measurement result sending button 206, and the display unit 207. The cursor leftward-move button 202 and the cursor rightward-move button 203 are used for moving the cursor to the left or to the right in order to select a desired selection candidate when a plurality of selection candidates are displayed on the input screen. The measurement start button 204 is used for causing the sensor 3 to start measuring the amount of urine or the concentration of a urine component. The excretion time information button 205 is used by the user at the measurement for entering the time information in relation to a bedtime activity such as "before-bedtime" or "first time after wake-up". To be more specific, when the excretion time information button 205 is pressed, selection candidates of the excretion time information, such as "before-bedtime", "first time after wake-up", and "second time after wake-up", are displayed on the display unit 207. With this state, the user can select from among the selection candidates using the cursor rightward-move button 203 or the cursor leftward-move button 202. The measurement result sending button 206 is used for causing the sending unit 217 to send the measurement result obtained by the sensor 3, the calculated daily urinary excretion, the health advice displayed on the display unit 207 corresponding to the daily urinary excretion, and the like, to a cellular telephone, a personal computer, or the like which is registered in advance.

(Method of measuring the amount of urine component)

Next, with reference to FIGS. 2 to 6, an explanation is given as to a method of measuring and analyzing the sodium amount excreted in the urine collected on an as needed basis and then estimating the amount of daily urinary sodium excretion, using this daily urinary excretion measurement apparatus 100.

First, the user collects the entire amount of urine 4 in a dedicated container (a beaker 1) as shown in FIG. 4 when urinating. When the user presses the measurement start button 204 on the control panel of the control unit 2, the CPU 211 starts a measurement control routine so that the urine measurement is started.

FIG. 6 is a flowchart showing the measurement control routine executed by the CPU 211 shown in FIG. 2.

When the measurement control routine is started, the CPU 211 carries out input of the measurement start time (the current time of measurement) detected by the timer unit 216 (step 311). Here, the user enters the excretion time information by pressing the excretion time information button 205 on the control panel.

Next, the CPU 211 executes the urine measurement processing (step 312). The measurement processing is performed respectively by the sodium sensor 302 and the urine amount sensor 301 included in the sensor 3. In the present embodiment, since both are measured according to the electrical conductivity, the sodium concentration and the urine amount are detected after the measurement of the electrical conductivity. The CPU 211 (the urine component calculation unit 411 shown in FIG. 3) calculates the urinary sodium amount by multiplying the sodium concentration and the urine amount together. Then, the urinary sodium amount is stored together with the excretion time information into the hard disk (the urine component accumulation unit 415 shown in FIG. 3).

On the basis of the data stored in the hard disk, the CPU 211 (the daily urinary excretion calculation unit 413 shown in FIG. 3) calculates the daily sodium excretion amount (step 313). Moreover, the CPU 211 (the daily urinary excretion calculation unit 413 shown in FIG. 3) compares the standard amount of the urinary sodium excretion previously stored in the hard disk (the advice storage unit 417) and the calculated daily urinary sodium excretion amount, and then calculates a difference from the standard amount (step 314). As the standard amount, an average value previously recommended by a medical institution or the like may be used. Alternatively, a target value of the user may be entered from the control panel. In the present case, the user enters the standard value by operating the buttons (the input unit 218) of the control panel. The result obtained from the comparison between the daily urinary sodium excretion amount and the standard amount is stored together with a measurement date and time into the hard disk (step 315).

Then, the urinary sodium amount, the daily urinary sodium excretion amount, and the result of the comparison with the standard value are displayed on the display unit 207 of the control panel (step 316). It should be noted that the result of the comparison with the standard value may be displayed when the user presses a result display button which is not illustrated. Here, in the case where the hard disk stores a health advice corresponding to the daily urinary sodium excretion amount or a result of the comparison with the standard value, the health advice can be displayed on the control panel together with the measurement result and the comparison result. As the health advice, various kinds of advice can be considered corresponding to the condition of the user. For example, there may be: an advice informing that excessive sodium intake can have an adverse effect on the health of the user; and a tip on limitation of salt intake in daily diet. Or, when the excretion amount is less than the standard value or the target value, a compliment message or the like may be displayed.

With the present configuration, the user can grasp the user's own daily urinary sodium excretion. Also, the user can be aware of how different the user's own salt intake is from the target value or the recommended value, which is useful in the self care related to the salt intake. Moreover, health advices and messages become an encouragement of the self care and a gathering of knowledge about proper healthcare.

To be more specific, when the user collects the before-bedtime urine and presses the measurement start button 204 on the control panel, the CPU 211 starts the measurement control routine so that the urine measurement is started. Following this, the user selects "before-bedtime urine" using the excretion time information button 205 on the control panel, the CPU 211 (the urine component calculation unit 411 and the daily urinary excretion calculation unit 413 shown in FIG. 3) judges that the excretion time information in the present case indicates that it is the before-bedtime urine and then uses the information for analysis. According to the procedure described in the first embodiment, the CPU calculates the before-bedtime urinary sodium excretion amount and stores the calculated amount together with "before-bedtime urine" as the excretion time information into the hard disk (the urine component accumulation unit 415).

Then, the user goes to bed, wakes up, and then urinates. As is the case with the before-bedtime urine, the user similarly collects the first morning urine after wake-up (the overnight urine) and presses the measurement start button 204. Thus, the CPU 211 starts the measurement control routine so that the urine measurement is started. Following this, the user selects "overnight urine" using the excretion time information button 205 on the control panel, the CPU 211 (the urine component calculation unit 411 and the daily urinary excretion calculation unit 413 shown in FIG. 3) judges that the excretion time information in the present case indicates the overnight urine and then uses the information for analysis. According to the procedure described in the first embodiment, the CPU 211 (the urine component calculation unit 411) calculates the overnight urinary sodium excretion amount and stores the calculated amount together with "overnight urine" as the excretion time information into the hard disk (the urine component accumulation unit 415).

The hard disk (the regression equation storage unit 416) stores a regression equation representing a correlation between: "before-bedtime urine" and "overnight urine"; and the daily urinary sodium excretion amount corresponding to an individual user. Thus, the CPU 211 estimates the daily urinary sodium excretion amount according to the regression equation based on the before-bedtime urinary sodium excretion and the overnight urinary sodium excretion.

FIG. 7 is a graph showing an example of the regression equation stored in the regression equation storage unit 416 shown in FIG. 3. Points shown in the graph of FIG. 7 are obtained by plotting measurement results shown in a table 1 below. In FIG. 7, the horizontal axis shows a spot urinary NaCl amount (g) whereas the vertical axis shows a daily NaCl excretion amount (g) included in the urine accumulated for one day. It should be noted that 8-hour correction shown in the table 1 and FIG. 7 will be explained later.

[Table 1]

| | Urinary NaCl amount (g) | | | | | | | | | | |
|---|---|---|---|---|---|---|---|---|---|---|---|
| Excretion time information | 1st day | 2nd day | 3rd day | 4th day | 5th day | 6th day | 7th day | 8th day | 9th day | 10th day | 11th day |
| Second time after wake-up | 1.07 | 2.66 | 4.37 | 1.74 | 2.06 | 1.83 | 2.71 | 2.01 | 1.89 | 1.97 | 1.28 |
| Before-bedtime | 1.01 | 1.03 | 2.09 | 3.8 | 1.09 | 2.69 | 3.48 | 2.35 | 1.17 | 0.24 | 0.67 |
| Overnight urine | 3.53 | 4.22 | 3.72 | 5.16 | 3.61 | 2.47 | 2.18 | 4.7 | 1.67 | 1.24 | 1.38 |

(continued)

| | Urinary NaCl amount (g) | | | | | | | | | | |
|---|---|---|---|---|---|---|---|---|---|---|---|
| Overnight urine (8-hour corrected) | 3.83 | 4.64 | 5.90 | 2.91 | 4.09 | 2.71 | 2.44 | 4.22 | 2.02 | 1.28 | 1.51 |
| Daily urine | 9.78 | 11.29 | 16.78 | 17.19 | 9.56 | 10.88 | 11.88 | 11.77 | 8.39 | 4.39 | 5.08 |
| Before-bedtime | 4.54 | 5.25 | 5.81 | 8.96 | 4.7 | 5.16 | 5.66 | 7.05 | 2.84 | 1.48 | 2.05 |
| + overnight | | | | | | | | | | | |
| Overnight + Second time after wake-up | 4.6 | 6.88 | 8.09 | 6.9 | 5.67 | 4.3 | 4.89 | 6.71 | 3.56 | 3.21 | 2.66 |
| Before-bedtime + overnight (8-hour corrected) | 4.84 | 5.67 | 7.99 | 6.71 | 5.18 | 5.4 | 5.92 | 6.57 | 3.19 | 1.52 | 2.18 |

On the basis of the results of the experiment in which a male in his thirties actually collected his urine for eleven days and the urinary NaCl amount is measured from the sodium concentration and the urine amount for each excretion, the table 1 records the urinary NaCl excretion amount (g) in association with the excretion time information for each measurement day. Based on this data, a regression equation indicated as an equation 1 is formulated. From this regression equation, the daily urinary sodium excretion amount is derived from a sum of the before-bedtime urinary sodium excretion amount and the overnight urinary sodium excretion amount. This regression equation is obtained, according to a least-squares method, from points indicated by open rhombuses plotted in FIG. 7 and is shown by a thick solid line. The points indicated by the open rhombuses are obtained by plotting the values of "Before-bedtime + overnight" of the sixth line of the table 1 as the horizontal axis and the values of "Daily urine" of the fifth line of the table 1 as the vertical axis.

[Equation 1]

Daily urinary sodium excretion amount (g) = 1.67 * (before-bedtime urinary sodium amount (g) + overnight urinary sodium amount (g)) + 2.53

The regression equation storage unit 416 stores the regression equation represented by the equation 1 which is obtained from the experimental results shown in the table 1, for example. The daily urinary excretion calculation unit 413 calculates the daily urinary excretion amount by substituting the before-bedtime urinary sodium excretion amount and the overnight urinary sodium excretion amount calculated by the urine component calculation unit 411 into the equation 1.

It should be noted that the first to fifth lines of the table 1 show data obtained by actual measurement and that the sixth to eighth lines show values obtained through calculation using the data shown in the first to fifth lines. For example, a sum of the before-bedtime urinary sodium excretion amount and the overnight urinary sodium excretion amount is calculated as "Before-bedtime + overnight" in the sixth line of the table 1. This value is obtained by adding the value of "Before-bedtime" of the second line and the value of "Overnight urine" of the third line of the table 1. In the table 1, for example, the before-bedtime urinary sodium excretion amount of the 1st measurement day is 1.01 g and the overnight urinary sodium excretion amount of the same 1st measurement day is 3.53 g. The value obtained by adding these values is 4.54 g which is shown in "Before-bedtime + overnight" of the sixth line of the table 1.

It should be noted that more than one regression equation may be stored by gender and age in the regression equation storage unit 416. In this case, the user may enter the user's gender and age using the input unit 218, so that the daily urinary excretion calculation unit 413 selects a regression equation corresponding to the gender and age of the user from the regression equation storage unit 416.

A table 2 shows correlations: between the urinary sodium excretion amount and the daily urinary sodium excretion amount; and between a combination of the urinary sodium excretion amounts measured within 24 hours and the daily urinary sodium excretion amount.

[Table 2]

| Target urine (excretion time condition) | Correlation with daily urinary sodium excretion ($R^2$) |
|---|---|
| Before-bedtime urinary sodium excretion + overnight urinary sodium excretion | 0.8108 |
| Overnight urinary sodium excretion + urinary sodium in second urine after wake-up | 0.7563 |
| Overnight urinary sodium excretion | 0.5908 |

As shown in the table 2, the correlation between "the sum of Before-bedtime urinary sodium excretion and overnight urinary sodium excretion" and "Daily urinary sodium excretion" ($R^2$ = 0.8108) of the present invention is higher than the conventional correlation between "Overnight urinary sodium excretion" and "Daily urinary sodium excretion" ($R^2$ = 0.5908). Thus, as to the correlation with the daily urinary sodium excretion, the amount estimated from the "before-bedtime urine" and the "overnight urine" accordingly to the present invention is higher than the daily urinary sodium excretion conventionally estimated only from the "overnight urine".

Moreover, the correlation between "the sum of Before-bedtime urinary sodium excretion amount and overnight urinary sodium excretion amount" and "Daily urinary sodium excretion amount" ($R^2$ = 0.8108) is higher than the correlation between "the sum of Overnight urinary sodium excretion amount + urinary sodium amount in second urine after wake-up" and "Daily urinary sodium excretion amount" ($R^2$ = 0.7563). Thus, it should be obvious that the daily excretion amount estimated from the urine collected in a plurality of times excreted within 24 hours with a sleep in between is higher in precision than that estimated from the urine collected in a plurality of times with no sleep in between.

(Second embodiment)

<8-hour correction is performed on overnight urinary sodium excretion amount>

FIG. 8 is a block diagram showing a configuration of a daily urinary excretion measurement apparatus 200 which calculates a daily urinary excretion amount though performing 8-hour correction on an overnight urinary sodium excretion amount. The present apparatus is different from the daily urinary excretion measurement apparatus 100 described above in that an 8-hour correction unit 712 performs 8-hour correction on the overnight urinary sodium excretion amount and that the 8-hour correction unit 712, a daily urinary excretion calculation unit 713, an excretion time information judgment unit 714, a urine component accumulation unit 715, a regression equation storage unit 716, a notification menu display unit 717, and a notification time table storage unit 718 are newly included. The notification menu display unit 717 and the display unit 207 of the daily urinary excretion measurement apparatus 200 corresponds to "a measurement notification unit which notifies a user of a timing at which the amount of the urine component is to be calculated by the urine component calculation unit."

The 8-hour correction unit 712 corresponds to "the daily urinary excretion calculation unit which corrects the amount of the overnight urine component by converting a period of time taken from an excretion time of the before-bedtime urine to an excretion time of the overnight urine into 8 hours, on the basis of the pieces of the excretion time information accumulated respectively in association with the amount of before-bedtime urine component and the amount of the overnight urine component." When the urinary sodium excretion calculated by the urine component calculation unit 411 is the overnight urinary sodium excretion amount, the 8-hour correction unit 712 performs the 8-hour correction on the calculated overnight urinary sodium excretion amount. To be more specific, the 8-hour correction unit 712 converts the calculated overnight urinary sodium excretion amount into an 8-hour overnight urinary sodium excretion amount by dividing the overnight urinary sodium excretion amount by a period of time taken from a time at which the before-bedtime urine of the previous day is measured to a time at which the overnight urine is measured and then multiplying this division result by 8 hours.

The daily urinary excretion calculation unit 713 corresponds to "the daily urinary excretion calculation unit calculates, in the calculating of the daily urinary excretion, the daily urinary excretion of the urine component using: the amount of the before-bedtime urine component calculated by the urine component calculation unit; and the amount of the overnight urine component calculated by the urine component calculation unit and corrected by the daily urinary excretion calculation unit in the performing of the 8-hour correction, on the basis of a correlation between: a sum of the before-bedtime urine component amount and the 8-hour corrected overnight urine component amount; and the total amount of the urine component included in the daily urine." To be more specific, the daily urinary sodium excretion amount is calculated using a regression equation which is stored in the regression equation storage unit 716 and which represents a correlation between: the before-bedtime urinary sodium excretion and the 8-hour corrected overnight urinary sodium excretion; and the daily urinary sodium excretion. In the graph of FIG. 7, the regression equation used here is indicated by a thick long dashed line which is obtained from the points indicated by "x" according to the least-squares method.

The excretion time information judgment unit 714 judges the excretion time information such as "overnight urine" or "before-bedtime urine" from a current time of measurement obtained by the timer unit 216 when the measurement is started.

The urine component accumulation unit 715 accumulates the overnight urinary sodium excretion on which the 8-hour correction has been performed by the 8-hour correction unit 712, in addition to the data accumulated by the urine component accumulation unit 315 of the first embodiment. This data corresponds to "Overnight urine (8-hour corrected)" of the fourth line of the table 1.

The regression equation storage unit 716 stores a regression equation which is obtained corresponding to an individual user as a result of previously measuring the data of the user. This regression equation represents a correlation between: the before-bedtime urinary sodium excretion and the 8-hour corrected overnight urinary sodium excretion; and the daily urinary sodium excretion.

When receiving an instruction from the input unit 218 that a time appropriate for measurement should be notified, the instruction being provided by the user through operating the buttons or the like of the control panel, the notification menu display unit 717 checks a measurement time appropriate for a next measurement, for example, by reference to a notification time table stored in the notification time table storage unit 718 and then causes the display unit 207 to display the measurement time.

The notification time table storage unit 718 stores the notification time table which is created in advance and shows times appropriate for measurement.

The second embodiment is different from the first embodiment in that the correlation between: the before-bedtime urinary sodium excretion and the 8-hour corrected overnight urinary sodium excretion; and the daily urinary sodium excretion is stored in the hard disk (the regression equation storage unit 716 in FIG. 8). To be more specific, the different point is that the regression equation to obtain the daily urinary sodium excretion, which is more appropriate for the user, is stored in the hard disk.

When the user collects the before-bedtime urine and presses the measurement start button 204 on the control panel, the CPU 211 starts the measurement control routine so that the urine measurement is started. When the measurement control routine is started, the CPU 211 carries out input of the current time of measurement detected by the timer unit 216. According to the procedure described in the first embodiment, the CPU 211 calculates the before-bedtime urinary sodium excretion and stores the calculated amount together with the current time of measurement into the hard disk (the urine component accumulation unit 715).

Then, the user goes to bed, wakes up, and then urinates. As is the case with the before-bedtime urine, the user similarly collects the first morning urine after wake-up (referred to as the overnight urine) and presses the measurement start button 204. Thus, the CPU 211 starts the measurement control routine so that the urine measurement is started. When the measurement control routine is started, the CPU 211 (the excretion time information judgment unit 714 in FIG. 8) carries out input of the current time of measurement detected by the timer unit 216. The CPU 211 (the excretion time information judgment unit 714) compares this current time of measurement with the previous current time of measurement stored in the hard disk (the urine component accumulation unit 715), judges that it is the overnight urine, and then uses the information for analysis. To be more specific, the judgment is made from that the urine related to the present measurement is excreted in the morning and that more than five hours have passed from the previous current time of measurement, for example. The CPU 211 (the excretion time information judgment unit 714) corrects the overnight urinary sodium excretion using a period of time passed since the previous excretion time.

(Overnight urinary excretion (n-hour correction)) = ((Overnight urinary sodium excretion) / (a period of time passed since the previous excretion time)) * n hours

In the present embodiment, a case where the overnight urinary excretion is corrected by eight hours is given as an example. The CPU 211 (the excretion time information judgment unit 714) determines that it is the before-bedtime urine from the previous time of data stored in the hard disk (the urine component accumulation unit 715) in consideration of the current time of measurement, and then uses this for analysis. The hard disk (the regression equation storage unit 716) stores a regression equation corresponding to an individual user that represents a correlation between: the before-bedtime urine and the overnight urine (8-hour corrected); and the daily urinary sodium excretion. The CPU 211 (the daily urinary excretion calculation unit 713) estimates the daily urinary sodium excretion according to the regression equation based on the before-bedtime urinary sodium excretion and the overnight urinary sodium excretion (8-hour corrected).

It should be noted that although the CPU 211 judges the excretion time information through the detection by the timer unit 216, the information provided from the excretion time information button on the control panel used by the user may be also used together.

Unlike the first embodiment, the regression equation representing the correlation between: the before-bedtime urinary sodium excretion and the 8-hour corrected overnight urinary sodium excretion; and the daily urinary sodium excretion is used in the present embodiment.

A table 3 shows a correlation between the urinary sodium excretion and the daily urinary sodium excretion on the basis of the results of the experiment in which a male in his thirties actually collected his urine for eleven days and the urinary sodium amount is measured from the sodium concentration and the urine amount for each urination. The table is obtained through analyzing the experimental results recorded in association with the urination times.

[Table 3]

| Target urine (excretion time condition) | Correlation with daily urinary sodium excretion ($R^2$) |
|---|---|
| Before-bedtime urinary sodium excretion + overnight urinary sodium excretion (8-hour corrected) | 0.8796 |
| Overnight urinary sodium excretion (8-hour corrected) | 0.4456 |

As shown in the table 3, the correlation between "the sum of Before-bedtime urinary sodium excretion and overnight urinary sodium excretion (8-hour corrected)" and "Daily urinary sodium excretion" ($R^2$ = 0.8796) of the present invention is higher than the conventional correlation between "Overnight urinary sodium excretion (8-hour corrected)" and "Daily urinary sodium excretion" ($R^2$ = 0.4456). Thus, as to the correlation with the daily urinary sodium excretion, the amount estimated from the "before-bedtime urine" and the "overnight urine (8-hour corrected)" accordingly to the present invention is higher than the daily urinary sodium excretion conventionally estimated only from the "overnight urine (8-hour corrected)".

Although the first and second embodiments have been explained using an example where the regression equation is formulated using eleven days of data, the number of days to be used is not particularly specified. Since each individual does not always take the same amount of salt intake and the amount may also vary according to seasons, it is preferable for the regression equation to be formulated and updated using data of more recent days. Also, the regression equation may be formulated for each group of days, such as commute and work days or holidays, which share similar patterns of sleep, diet, exercise, and activities.

FIG. 9 is a diagram of an external view showing an appearance of a control panel of the daily urinary excretion measurement apparatus 200 of the second embodiment. The daily urinary excretion measurement apparatus 200 of the present embodiment includes a measurement start button 204, a display unit 207, excretion time information buttons 230, a result display button 240, cursor move buttons 250, and a measurement notification menu button 260. The excretion time information buttons 230 include a time button used by the user to manually enter the current time of measurement, in addition to buttons to receive the excretion time information as "before-bedtime urine" or "overnight urine". The result display button, when pressed by the user, provides a signal to the daily urinary excretion calculation unit 713 to instruct that the daily urinary sodium excretion which is the calculation result given by the daily urinary excretion calculation unit 713 and the corresponding health advice should be displayed. The cursor move buttons 250 are used for scrolling the health advices and the like displayed on the display unit 207 and for moving the cursor in order to select one out of a plurality of selection candidates. The measurement notification menu button 260 is used for instructing the notification menu display unit 717 to display a urination time appropriate for a next measurement, in order to remind the user to measure the urine related to the excretion time information having a high correlation with the daily urinary sodium amount.

The daily urinary excretion measurement apparatus 200 is different from the daily urinary excretion measurement apparatus 100 of the first embodiment in that the display unit 207 of the control panel is larger and that a display screen for a measurement notification menu notifying about the measurement timing can be displayed on the display unit 207. This measurement notification menu reminds the user to measure the urine at the time represented by the excretion time information having a high correlation with the daily urinary sodium amount. In the present embodiment, considering that the biological rhythms of urinary sodium excretion are different between a time of day for activities including having meals and exercising and a time of day for sleeping, the before-bedtime urine is used as a urine sample of the time of day for activities whereas the overnight urine is used as a sample of the time of day for sleeping. Thus, when the user selects the measurement notification menu, notification by a sound through an alarm generation or notification by light, for example, is made at the time represented by the excretion time information having a high correlation with the daily urinary sodium amount, that is, the notifications are made at a time to go to bed and a time to wake up so as to alert and remind the user of the measurement. The time to go to bed and the time to wake up may be entered by the user, or the notifications are made at times of day generally estimated as the bedtime and the wake-up time. Alternatively, as a standby display of the control panel, the excretion time information having a high correlation with the daily urinary sodium amount may be displayed.

With the present configuration, it becomes easy to carry out the measurement at an excretion time having a high correlation with the daily urinary sodium amount, so that the daily excretion amount can be accordingly estimated with a high degree of accuracy.

(Third embodiment)

<Formulation of regression equation for each individual>

FIG. 10 is a block diagram showing a configuration of a daily urinary excretion measurement apparatus 300 which formulates a regression equation for each user. The daily urinary excretion measurement apparatus 300 of the third embodiment accumulates the before-bedtime urinary sodium excretion, the 8-hour corrected overnight urinary sodium excretion, and the daily urinary sodium excretion corresponding to the sum of the above two for at least three days, and formulates a regression equation for each user according to the least-squares method as shown in the graph of FIG. 7. After formulating the regression equation, the daily urinary excretion measurement apparatus calculates the daily urinary sodium excretion using the formulated regression equation. The daily urinary excretion measurement apparatus 300 includes an advice storage unit 417, an 8-hour correction unit 712, a daily urinary excretion calculation unit 713, a urine component accumulation unit 715, a regression equation storage unit 716, a urine component calculation unit 911, and a regression equation calculation unit 914.

The daily urinary excretion measurement 300 corresponds to "the daily urinary excretion measurement apparatus further including: a urine component accumulation unit which accumulates the amounts of the before-bedtime urine component and the amounts of the overnight urine component of at least three days together with the respective corresponding daily urinary excretions; and a correlation calculation unit which calculates, from: a sum of the amount of the before-bedtime urine component and the amount of the overnight urine component; and the corresponding daily urinary excretion accumulated in the urine component accumulation unit, a correlation between: the sum of the amount of the before-bedtime urine component and the amount of the overnight urine component; and the corresponding daily urinary excretion according to a linear approximation." The urine component accumulation unit 715 corresponds to the "urine component accumulation unit"; the regression equation corresponds to the "correlation"; and the regression equation calculation unit 914 corresponds to the "correlation calculation unit".

The urine component calculation unit 911 and the regression equation calculation unit 914 are added as new components to the daily urinary excretion measurement apparatus 300. Thus, operations performed by the urine component calculation unit 911 and the regression equation calculation unit 914 are explained in detail with reference to a flowchart shown in FIG. 11. FIG. 11 is a flowchart showing a flow of processing performed when the daily urinary excretion measurement apparatus formulates a regression equation for each user.

When the user collects urine and presses a regression equation formulation button, which is not illustrated, on the control panel that is the input unit 218, the CPU 211 starts a regression equation formulation routine so that the urine measurement is started. When the regression equation formulation routine is started, the CPU 211 (the urine component calculation unit 911) carries out input of the current time of measurement detected by the timer unit 216 (S801). Then, the user enters the excretion time information, such as "before-bedtime urine" or "overnight urine". When the urine amount and the sodium concentration are measured by the sensor 3 (S802), the CPU 211 (the urine component calculation unit 911) calculates the urinary sodium excretion amount (S803) and stores the calculated amount together with the current time of measurement and the excretion time information into the hard disk (the urine component accumulation unit 715) according to the procedure described in the first embodiment (S804). When the entered excretion time information is the "overnight urine", the 8-hour correction unit 712 performs the 8-hour correction on the calculated urine component amount as in the case of the second embodiment, and stores the 8-hour corrected amount together with the current time of measurement and the excretion time information "8-hour corrected overnight urine" into the hard disk (the urine component accumulation unit 715). The urine component calculation unit 911 (and the 8-hour correction unit 712) repeats the processing from Step S801 to Step S804, and accumulates (A) the measured urine amounts, sodium concentrations, urinary sodium amounts, and the 8-hour corrected overnight urinary sodium amount of the day into the hard disk (the urine component accumulation unit 715) in association with the current times of measurement and the excretion time information (S805). By calculating the sum of the urinary sodium amounts of the whole day accumulated in the urine component accumulation unit 715, the urine component calculation unit 911 calculates the daily urinary sodium excretion amount (S806) and accumulates the calculated daily urinary sodium excretion amount and (A) as the excretion data of the whole day into the urine component accumulation unit 715 (S807).

The urine component calculation unit 911 and the 8-hour correction unit 712 repeat the processing from Step S801 to Step S807, and accumulate the excretion data of the whole day into the hard disk (the urine component accumulation unit 715) in association with the current times of measurement and the excretion time information for several days, preferably five days or at least three days (S808).

The regression equation calculation unit 914 plots, for each measurement day, a plurality of combinations of the urinary sodium excretion amounts (especially the sum of the before-bedtime urinary sodium excretion and the 8-hour corrected overnight urinary sodium excretion) accumulated in association with the excretion time information and the daily urinary sodium excretion amount of the current day calculated in Step S806, and thus makes a graph as shown in FIG. 7 (S809). The regression equation calculation unit 914 calculates the regression equation from the points plotted on the graph, according to the least-squares method (S810). The regression equation calculation unit 914 stores the calculated regression equation into the regression equation storage unit 716 (S811).

According to the third embodiment as described so far, the user measures all the urine excreted in the day using the daily urinary excretion measurement apparatus 300 for several days, so that an individualized regression equation is automatically formulated for each user. Thus, the user does not need to go to a hospital or a health center to have a regression equation formulated, and can formulate an individualized regression equation by the user himself or herself. In addition, as compared to the case where the daily urinary excretion amount is calculated using a standard regression equation classified by gender and age, a more accurate daily urinary excretion amount can be calculated through calculation of the daily urinary excretion amount using the individually-formulated regression equation. This, as a result, can provide a more appropriate health advice.

In the stated embodiment, the user accumulates the excretion data of one whole day and calculates the daily urinary sodium excretion amount by summing up the urinary sodium amounts included in the accumulated excretion data. However, the user does not need to accumulate the excretion data of one whole day but, for example, the regression equation may be formulated by measuring only the before-bedtime urinary sodium excretion and the 8-hour corrected overnight urinary sodium excretion for several days although the accuracy of the regression equation is thus lowered. In this case, the daily urinary sodium excretion amount calculated by the daily urinary excretion calculation unit 713 using the standard regression equation classified by gender and age is used. With this, the user does not need to measure the whole day's urine and only measures a couple of times including the measurements of the before-bedtime urinary excretion and the overnight urine, thereby obtaining the customized regression equation of the user's own. Moreover, amendments may be made to the regression equation with each measurement, so that the regression equation which is more appropriate for the individual user can be obtained.

(Fourth embodiment)

<Creatinine correction on urine amount>

A spot urine sample collected in one urine sampling is often concentrated or diluted due to fluid intake or perspiration. For this reason, abnormalities of urine components may be overrated or underrated when an evaluation is made on the basis of the concentration. To address this problem, there are methods such as: a method of measuring a urine component and a urine amount at the same time so as to calculate a daily excretion amount; and a method of measuring a urine component and a urinary creatinine at the same time so as to make an evaluation according to a ratio between the urine component and creatinine. The explanation has already been given in the above first to third embodiments as to the method of measuring the urine component and the urine amount at the same time so as to calculate the daily excretion amount.

The following is a detailed explanation about the method of measuring the concentration of a urine component and the concentration of urinary creatinine at the same time so as to make an evaluation according to the urine-component/creatinine ratio. Creatinine is a dehydration product of creatine, and is not biologically active. Creatinine is produced directly from creatine phosphate and through dehydration of creatine in muscle and nerves within a living body, and is secreted into blood. After renal glomerular filtration, creatinine is excreted in urine with almost no reabsorption. The amount of creatinine production is proportional to a total amount of creatine of muscle (that is, the total amount of muscle). Thus, creatine is produced at a constant rate per kilogram of body weight in the case of an adult. Moreover, since the daily urinary creatinine excretion amount is little affected by dietary factors or the urine amount, creatine is produced at a constant rate within an individual organism as long as there is no shock or loss of blood. On account of this, the urinary creatinine amount is measured for the purpose of performing correction on the urine amount of the urine component excretion amount.

Creatinine correction is frequently used in clinical practice since a urine component can be evaluated after correcting effects caused by concentrated or diluted urine. The daily urine component excretion can also be calculated by multiplying the urine-component/creatinine ratio by the daily urinary creatinine excretion. Thus, a method of estimating the daily component excretion from a component amount in the spot urine sample is under consideration.

A daily urinary Cre excretion exhibits relatively less fluctuation within an individual organism and is said to have a high correlation with age and physical size. With attention being given to this point, Tanaka et al. (Non-patent document: H. Ueshima, et al.: A simple method to estimate populational 24-h urinary sodium and potassium excretion using a casual urine specimen. Journal of Human Hypertension, 16, 97-103 (2002)) and Kawasaki et al. have formulated the equation of estimating a daily urinary Cre excretion through multiple regression analysis on Japanese people, whereby age, height, and weight are independent variables and a measured value of the daily urinary Cre excretion is a dependent variable. The estimation equation formulated by Tanaka et al. is described below.

$$\text{Predicted value of 24-h urinary Cre excretion (PRCr)} = -2.04 * (\text{age}) + 14.89 * (\text{weight (kg)}) + 16.14 * (\text{height (cm)}) - 2244.55 \text{ (mg/day)}$$

Moreover, Tanaka et al. and Kawasaki et al. have formulated the equation of estimating the amount of 24-h Na excretion. For formulating the equation, a predicted value XNa of 24-h urinary Na excretion is calculated by multiplying a ratio between a spot urine Na and Cre by the predicted value PRCr of 24-h urinary Cre excretion, and then the calculated predicted value XNa of 24-h urinary Na excretion and the measured value are compared. The equation of estimating the 24-h Na excretion formulated by Tanaka et al. is described below. Note that SU in the following equation represents the spot urine sample.

$$\text{Predicted value of 24-h urinary Na excretion (XNa)} = \{(\text{Na concentration in SU}) / (\text{Cre concentration in SU})\} * (\text{PRCr})$$

$$\text{24-h urinary Na excretion (mEq / day)} = 21.98 * XNa^{0.392}$$

When the daily urinary excretion measurement method based on the urine amount measurement is employed, the user needs to collect all the urine excreted each time and measure the amount of urine. On the other hand, when creatinine correction is employed, a sensor for measuring the creatinine concentration is used in place of the sensor for measuring the amount of urine and, therefore, the amount of urine is not measured. Instead, the concentration of creatinine in the spot urine sample is measured and, from this result, a daily creatinine excretion is estimated and the urine component amount is accordingly corrected. Thus, the user only needs to collect a part of urine excreted during urination and can be freed from the requirement of colleting all the urine. As a result, the daily urinary excretion can be calculated more easily as compared with the method whereby the daily urinary excretion is calculated from the concentration of the urine component in the spot urine sample and the amount of urine. Accordingly, when the daily urinary excretion measurement apparatus of the fourth embodiment is used, the urine can also be collected using a urine collection apparatus which is included in a toilet or a bidet apparatus.

It should be noted that although the equation formulated by Tanaka et al. is used as the creatinine correction method in the fourth embodiment, the present invention is not limited to this. As long as the daily urinary excretion of a component is calculated from the urinary amount of this component in the spot urine sample using the concentration of creatinine in the spot urine sample (or, from the ratio between the urine component and creatinine), any equation can be used in the same way. For example, Tanaka et al. used the above-described estimation equation where age, height, and weight are independent variables. However, an estimation equation where gender is an independent variable may be used. Additionally, as another method, a table instead of the estimation equations described above may be stored that shows: one of or a combination of age, height, weight, and gender; and a corresponding daily creatinine excretion. In this case, the daily creatinine excretion of a subject may be read from the table, and the daily urinary excretion may be calculated by multiplying the read excretion by the urine-component/creatinine ratio for each spot urine sample.

FIG. 12 is a diagram showing a hardware configuration of a daily urinary excretion measurement apparatus 400 of the fourth embodiment. The daily urinary excretion measurement apparatus 400 of the fourth embodiment includes a sensor 3 which has a creatinine sensor in place of the urine amount sensor of the first embodiment. To be more specific, the daily urinary excretion measurement apparatus 400 measures the concentration of creatinine and the concentration of a urine component for each of a plurality of spot urine samples collected within a given period of time, so that an average daily total amount of the urine component excretion (a daily amount measured since the urination time of the first morning urine after wake up, for example) within the given period of time is automatically calculated. The daily urinary excretion measurement apparatus 400 includes a control unit 2 and the sensor 3. The control unit 2 includes a display unit 207, a CPU 211, a memory 215, a timer unit 216, a sending unit 217, and an input unit 218, which are interconnected via buses. The sensor 3 includes a creatinine sensor 303 and a sodium sensor 302.

The display unit 207 includes a liquid crystal display. However, it is not especially limited to this as long as it displays measurement data and analysis data. The display unit 207 displays: a data input screen; an output screen for displaying the measurement data and the analysis data; and an instruction screen for displaying a measurement instruction. To be more specific, the display unit 207 displays the measured concentration of creatinine, the measured concentration of a urine component, the amount of the urine component represented by the urine-component/creatinine ratio, and the calculated daily urinary excretion. In addition, the display unit 207 corresponds to "a display unit which displays an advice when the comparison unit judges that the daily urinary excretion exceeds the standard amount", and displays a health advice on the basis of the calculated daily urinary excretion. Moreover, the display unit 207 displays a message screen for notifying the user of a preferable timing for measurement. The CPU 211 is responsible for controlling the entire control unit 2. In accordance with a program stored in a ROM, the CPU 211 performs secondary processing on the measurement data, such as processing of urine measurements using the sensors, processing of estimating a daily urinary excretion using the measured urinary sodium concentration and the measured urinary creatinine concentration; and processing of estimating an average daily urinary excretion from the measurement data obtained from a plurality of spot urine samples. Moreover, the CPU 211 receives an instruction from the user and displays corresponding information on the display unit 207 by exchanging signals with the input unit 218 and a touch panel included in a control panel.

The memory 215 includes a RAM, a ROM, an IC memory, and a hard disk. For example, the memory 215 previously stores, in the ROM or the like, a correlation between the concentration of creatinine in a spot urine sample and a daily urinary excretion amount. Also, the readable/writable large-capacity memory 215, which is realized by the hard disk, the IC memory, and the like, accumulates the measurement data, such as the measured concentration of creatinine, the measured concentration of the urine component, and the amount of the urine component, in association with the excretion time information for each measurement.

The timer unit 216 is a timer or the like, and detects urination time information.

The sending unit 217 sends a measurement result, a health advice, or the like to an external personal computer, a cellular telephone, a PDA, or the like which is registered in advance by the user.

The input unit 218 includes a various kinds of buttons provided on the control unit 2 and a touch panel provided on the display unit 207.

It should be noted that, although illustration is omitted, the daily urinary excretion measurement apparatus 100 of the present embodiment includes an input/output port and so can perform input and output of data into and from a flexible disk and the IC memory via a flexible disk drive or an IC memory drive externally connected to the input/output port.

The creatinine sensor 303 measures the concentration of creatinine in the urine sucked into the sensor. To be more specific, creatinine is measured according to an enzyme method. Inside the sensor, creatinine amidohydrolase, creatinine amidinohydrolase, and sarcosine oxidase which are enzymes for measuring creatinine, and a dye are dried and held. Then, the concentration of creatinine is measured through a judgment of a color change of the dye (Reference document: Japanese Unexamined Patent Application Publication No. 54-151095).

It should be noted that the method of measuring creatinine is not limited to the enzyme method used for measuring creatinine in the present embodiment. As another creatinine measurement method according to the enzyme method, a method of detecting ammonia using creatinine deaminase (Reference document: Japanese Unexamined Patent Application Publication No. 2001-512692) may be used here. Alternatively, another easy method of measuring the concentration of urinary creatinine may be used if any, as long as the concentration of creatinine in a spot urine sample can be measured.

The sodium sensor 302 measures a urinary sodium concentration of the urine sucked into the sensor 3. The sodium sensor 302 measures the urinary sodium concentration according to an electrode method employing an electrical conductivity method. The measurement of the electrical conductivity can be realized by measuring impedance between predetermined terminals. Since having no moving parts, the configuration is simple and thus its operation reliability is high. The electrical conductivity of urine generally depends on the sodium concentration. On account of this, a calibration curve may be previously obtained from a correlation between the urinary sodium concentration and the electrical conductivity, and then the sodium concentration can be measured using an inexpensive sensor which has two electrodes for measuring the electrical conductivity. It should be noted that when the sodium concentration is measured on the basis of the electrical conductivity, correction may be performed in order to remove an error caused by potassium, magnesium, and the like other than sodium.

FIG. 13 is a function block diagram showing major units included in the daily urinary excretion measurement apparatus 400 which is realized by the CPU 211 and the memory 215 shown in FIG. 12. As shown in this diagram, the daily urinary excretion measurement apparatus 400 includes a urine component calculation unit 411, an average daily urinary excretion calculation unit 418, a urine component accumulation unit 415, a regression equation storage unit 416, and an advice storage unit 417.

The daily urinary excretion measurement method employed by the daily urinary excretion measurement apparatus 400 corresponds to "the daily urinary excretion measurement method wherein, in the calculating of the amount of the urine component, the urine component calculation unit calculates a ratio of the urine component to creatinine in the spot urine sample as the amount of the urine component included in the spot urine sample, from a component concentration of the spot urine sample measured by a component sensor and a creatinine concentration of the spot urine sample measured by a creatinine sensor, and in the calculating of the daily urinary excretion, the daily urinary excretion calculation unit calculates the daily urinary excretion of the urine component, using: the creatinine ratio which is calculated as the amount of the urine component by the urine component calculation unit; and an amount of daily creatinine excretion held in advance by the urine component calculation unit. Here, the urine component calculation unit 411 corresponds to "the urine component calculation unit which calculates a ratio of the urine component to creatinine in the spot urine sample as the amount of the urine component included in the spot urine sample, from the component concentration of the spot urine sample measured by the component sensor and the creatinine concentration of the spot urine sample measured by the creatinine sensor." The average daily urinary excretion calculation unit 418 corresponds to "the daily urinary excretion calculation unit which calculates, in the calculating of the daily urinary excretion, the daily urinary excretion of the urine component using: the creatinine ratio which is calculated as the amount of the urine component by the urine component calculation unit; and the amount of daily creatinine excretion held in advance by the urine component calculation unit."

Moreover, the daily urinary excretion measurement method employed by the daily urinary excretion measurement apparatus 400 corresponds to "the daily urinary excretion measurement method wherein the daily urinary excretion calculation unit calculates the daily urinary excretion using the amounts of the urine component of at least three but not exceeding fourteen spot urine samples calculated by the urine component calculation unit within the predetermined period of time." The average daily urinary excretion calculation unit 418 corresponds to the "daily urinary excretion calculation unit".

From the creatinine concentration received from the creatinine sensor 303 and the sodium concentration received from the sodium sensor 302, the urine component calculation unit 411 calculates a ratio between the amount of sodium and the amount of creatinine in the spot urine sample. Moreover, the urine component calculation unit 411 accumulates, into the urine component accumulation unit 415, the received creatinine concentration, the received sodium concentration, and the calculated sodium/creatinine ratio together with the current time of measurement obtained from the timer unit 216 and the excretion time information received from the input unit 218.

When the urine component calculation unit 411 calculates the spot-urine sodium amount, the average daily urinary excretion calculation unit 418 reads the most recent five sodium/creatinine ratios which were measured within a week from this current measurement time from the urine component accumulation unit 415. Next, the average daily urinary excretion calculation unit 418 calculates the daily urinary sodium excretion amount by substituting these read six sodium/creatinine ratios into the regression equations (the above three equations by Tanaka et al.). Moreover, the average daily urinary excretion calculation unit 418 calculates the user's average daily urinary sodium excretion of the past week by calculating an average of the calculated six sets of measurement data, that is, an average of the daily urinary sodium excretion amounts. Furthermore, the average daily urinary excretion calculation unit 418 reads an advice corresponding to the calculated average daily urinary excretion amount from the advice storage unit 417 and causes the display unit 207 to display the advice. Following the instruction received from the input unit 218, the daily urinary excretion calculation unit 413 sends information including the calculated daily urinary excretion amount and the corresponding advice to the sending unit 217.

For each time of spot urine measurement, the urine component accumulation unit 415 accumulates the current time of measurement, the excretion time information, the creatinine concentration, and the sodium concentration. When the sodium/creatinine ratio of the spot urine sample, the average daily urinary excretion amount, the average daily urinary sodium excretion amount, and a difference from a standard amount are calculated, these are also accumulated. The regression equation storage unit 416 stores a regression equation, which is previously calculated, that shows a correlation between each sodium/creatinine ratio of a plurality of spot urine samples and the average urinary sodium excretion amount. The advice storage unit 417 stores a predetermined health advice corresponding to a standard amount of the average daily urinary sodium excretion or a predetermined health advice corresponding to a difference from the standard amount.

(Method of measuring the amount of urine component)

Next, with reference to FIGS. 2 to 6, an explanation is given as to a method of measuring and analyzing the sodium amount excreted in the urine collected on an as needed basis and then estimating the amount of daily urinary sodium excretion, using this daily urinary excretion measurement apparatus 400.

First, the user collects a part of the urine when urinating and dips a sensor part placed on the measurement apparatus into the collected urine. When the user presses the measurement start button 204 on the control panel of the control unit 2, the CPU 211 starts a measurement control routine so that the urine measurement is started.

FIG. 6 is a flowchart showing the measurement control routine executed by the CPU 211 shown in FIG. 2.

When the measurement control routine is started, the CPU 211 carries out input of the measurement start time (the current time of measurement) detected by the timer unit 216 (step 311). Here, the user enters the excretion time information by pressing the excretion time information button 205 on the control panel.

Next, the CPU 211 executes the urine measurement processing (step 312). The measurement processing is performed by the sodium sensor 302 and the creatinine sensor 303 included in the sensor 3. In the present embodiment, the sodium concentration is detected after the measurement of the electrical conductivity and the creatinine concentration is detected after the optical measurement. The CPU 211 (the urine component calculation unit 411 shown in FIG. 3) calculates the urinary sodium excretion amount according to the equation formulated by Tanaka et al. using the sodium concentration, the creatinine concentration, and the previously received values of age, height, and weight of the user. Then, the average daily urinary sodium excretion amount is calculated by calculating an average value of total six sets of data which includes five results of the daily urinary sodium excretion amounts obtained from the overnight urine or the before-bedtime urine measured within the past week from the time of the current measurement and one result of the daily urinary sodium excretion amount measured this time. This value represents the average sodium intake of the past nearly one week from the date of the measurement this time.

It should be noted that when the measurement has not been performed a specified number of times (six times in total in the present case) within a given period of time (one week in the present case) from the current measurement time, the display unit of the measurement apparatus displays: that the average amount of intake cannot be calculated; a guide indicating how many times of measurement are left; and a comment appreciating that the current measurement has been carried out. This can encourage the user of the measurement apparatus to perform the measurement and to continue the lifestyle improvement (salt reduction) that follows.

Moreover, the CPU 211 (the daily urinary excretion calculation unit 413 shown in FIG. 3) compares the standard amount of the urinary sodium excretion previously stored in the hard disk (the advice storage unit 417) and the calculated average daily urinary sodium excretion amount, and then calculates a difference from the standard amount (step 314). As the standard amount, an average value previously recommended by a medical institution or the like may be used. Alternatively, a target value of the user may be entered from the control panel. In the present case, the user enters the standard value by operating the buttons (the input unit 218) of the control panel. The result obtained from the comparison between the daily urinary sodium excretion amount and the standard amount is stored together with a measurement date and time into the hard disk (step 315).

Then, the urinary sodium amount, the daily urinary sodium excretion amount, and the result of the comparison with the standard value are displayed on the display unit 207 of the control panel (step 316). It should be noted that the result of the comparison with the standard value may be displayed when the user presses a result display button which is not illustrated. Here, in the case where the hard disk stores a health advice corresponding to the average daily urinary sodium excretion amount or a result of the comparison with the standard value, the health advice can be displayed on the control panel together with the measurement result and the comparison result. As the health advice, various kinds of advice can be considered corresponding to the condition of the user. For example, there may be: an advice informing that excessive sodium intake can have an adverse effect on the health of the user; and a tip on limitation of salt intake in daily diet. Or, when the excretion amount is less than the standard value or the target value, a compliment message or the like may be displayed.

With the present configuration, the user can grasp the user's own average daily urinary sodium excretion. Also, the user can be aware of how different the user's own salt intake is from the target value or the recommended value, which is useful in the self care related to the salt intake. Moreover, health advices and messages become an encouragement of the self care and a gathering of knowledge about proper healthcare.

In the case of the present configuration, an explanation has been given as to a case where the number of measurements of the spot urine samples including the overnight urine and/or the before-bedtime urine is six within one week.

Next, an assessment was made as to whether, when the amount of daily salt intake was limited to a certain degree, the limit degree of salt intake can be judged using the measurement apparatus having the present configuration. The assessment result is shown below. For six days, adult males and females (eight in total) consumed diets, with the daily salt intake being limited to a certain value. Then, measurements were carried out using the measurement apparatus described in the fourth embodiment for each of a high salt intake period and a low salt intake period. The overnight urine and the before-bedtime urine were measured every day. The assessment as to whether the limit degree of salt intake can be judged is about whether a difference between the high salt intake period and the low salt intake period can be discerned. In the present case, the difference of the salt intake amounts between the periods was 4 g salt per day. FIG. 14 is a graph showing the number of period-difference detections in the case of using an average value of data obtained through a plurality of measurements that include both the overnight urine measurement and the before bedtime urine measurement. It should be noted that, in the graphs shown in FIG. 14 as well as in FIGS. 15 and 16 described below, the horizontal axis shows the number of consecutive measurements within a week. The number of period-difference detections shows the number of users, out of the eight users, who could detect the difference between the high salt intake period and the low salt intake period using the average daily urinary excretion measured for each of the periods. As a result, when six or more measurements were carried out, six or more out of the eight users detected the difference in the salt intake amount. Also, when four to five measurements were carried out, four users, which is half of the eight users, detected the difference in the salt intake amount.

FIG. 15 is a graph showing the number of period-difference detections in the case of using an average value of data obtained through a plurality of measurements that include only the overnight urine measurements. As shown in FIG. 15, when only the overnight urine (the morning urine) was measured, five users out of the eight could detect the difference using the average of four or more measurements. Also, using the average of three measurements, four users, which is half of the eight users, could detect the difference. FIG. 16 is a graph showing the number of period-difference detections in the case of using an average value of data obtained through a plurality of measurements that include only the before bedtime urine measurements. As shown in FIG. 16, when only the before-bedtime urine (night urine) was measured, four or more users out of the eight could detect the difference through four or more measurements.

In this way, the average intake amount can be grasped for each period by measuring the daily urinary excretion amount a plurality of times. Moreover, when the intake amount of the component to be excreted in the urine is different for each period, this difference can be detected with a high degree of accuracy using the measurement apparatus described in the present embodiment.

Furthermore, as explained in the fourth embodiment, after the predetermined number (six, for example) of measurements are completed within the given period of time (a week, for example), the display unit 207 may display the average of the daily urinary sodium excretion amounts measured by the daily urinary excretion measurement apparatus and the advice. With this, the measurement result with a higher degree of accuracy as well as a more appropriate advice can be provided to the user. Here, the display unit 207 corresponds to "a display unit which displays the daily urinary excretion calculated by the daily urinary excretion calculation unit only after the urine component calculation unit calculates the amount of the urine component for each of the plurality of spot urine samples collected within the predetermined period of time."

As described so far, according to the daily urinary excretion measurement apparatus explained in the present embodiment, the average daily urinary excretion amount within the given period of time can be obtained as a result of using three or more of the spot-urine measurement results. From this result, a difference in the intake amount can be detected between the periods during which the intake amounts are different. This can be used as an effect evaluation tool for guidance on salt reduction in a field of treatment or prevention of lifestyle-related diseases.

In the present embodiment, creatinine correction has been used, so that a degree of accuracy of measurement is lowered as compared to the case where the daily urinary excretion is calculated on the basis of the measurement of the urine component amount. However, convenience for the user of the measurement apparatus is greatly improved and, therefore, it can be said to be an easier measurement method to calculate the daily urinary excretion amount based on creatinine correction.

It should be noted that the urine component measurement unit is not particularly limited as long as it can measure the amount of a component excreted in the urine. For example, a method whereby the amount of a component excreted in urine is measured using a combination of a unit which measures the amount of urine and a unit which measures the concentration of the urine component may be used.

Moreover, although urinary sodium has been used as an example of a urine component in the above embodiments, a urine component whose daily urinary excretion amount can be measured by the daily urinary excretion measurement apparatus of the present invention is not limited to the urinary sodium. As a urine component from which a health condition of the user can be detected, protein, salt, uric acid, etc. can be used. As a unit for detecting the concentration of a urine component, various kinds of methods may be used depending on a subject of measurement.

Protein is also called urinary albumin. The concentration of urinary albumin is used as a barometer of assessing kidney function. Especially for diabetic renal disease which is developed from the progression of diabetes, it is considered to be important to detect a trace of albumin that is excreted during the early stage, in order to cause early detection. Various kinds of units can be used for detecting albumin, and an immunization method or an immunoturbidimetric method using anti-albumin antibody which phenomenally combines albumin can be used, for example.

Salt is known to cause hypertension and stomach cancer. Thus, it is desirable to perform management so that the amount of salt intake stays within an appropriate value for prevention and treatment of these diseases. The amount of sodium excreted in the urine is approximately the same as the amount of sodium intake. For this reason, the daily sodium intake can be estimated by measuring the daily amount of sodium excreted in the urine. As a unit for detecting the urinary sodium, various kinds of methods can be used. For example, an electrode method based on an electrical conductivity method; a method using selective electrodes which selectively reacts with sodium ion; and a method of detecting chloride ion according to a coulometric titration method or an ion selective electrode method can be used.

Uric acid is a substance known to cause diseases including gout. It is said that about a little less than 80% of the daily produced uric acid is excreted in the urine. On account of this, by measuring uric acid in the urine, the uric acid level of the body can be estimated. Thus, this can be used for prevention and treatment of gout and other diseases. As a method of detecting uric acid, various kinds of methods can be used, such as an electrode method and a method of detecting the concentration of uric acid on the basis of a color of solution after uric acid is broken down by uricase.

The excretion time information specifies a time at which the urine is excreted. The information includes a date and a time of day when the urine is excreted, for example. The excretion time information may also include: time frames, such as early morning, daytime, midnight, morning, and afternoon; seasons, such as spring, summer, fall, and winter; times related to daily life, such as before-meal, after-meal, before-exercise, after-exercise, before-bedtime, and after wake-up; and the number of urinations after wake-up, such as a first time after wake-up, a second time after wake-up....

The excretion time information can be detected by a timer, for example, that has a timer unit. When a calendar function is included, a date and time of the measurement may also be detected. With this function, in addition to days of the week obviously, weekdays and holidays can also be distinguished, so that lifestyle patterns including sleep, diet, exercise, and activities can be inferred from analogy.

As an input method of the excretion time information, the CPU or the like may execute the input in accordance with a stored program, or the user may directly perform the input. The urine component excretion is known to have a biological rhythm, and the amount of excretion largely depends on environmental factors including a time of urination, details of activities, lifestyle, and climate. Thus, conditions in which the urine is excreted are important when the urinary excretion is considered. For this reason, the measured amount of urinary excretion is stored and analyzed together with the excretion time information, which is an important measurement condition. A type of the storage unit is not particularly limited, and a nonvolatile memory such as a hard disk or movable record media such as a CD-ROM and an SD-card may be used for example. Although the urine component excretion is known to vary considerably from individual to individual, the excretion of one individual also greatly fluctuates throughout the day. Therefore, by analyzing the several days of measurement data stored in the storage unit, the measurement result which more reflects the health condition of the user can be obtained.

Various kinds of methods can be used for judging from the excretion time information whether it is before or after bedtime. The methods include: a method using the timer unit; and a method causing the user to press a button of the excretion time information related to the bedtime activities, such as "Before-bedtime" or "First time after wake-up".

As another method of judging from the excretion time information whether the excretion time is before or after bedtime, the method of performing the input of the bedtime information is used for the measurement apparatus. However, note that the present invention is not limited to this. For example, the measurement apparatus may store an average sleep time pattern (an average bedtime and an average wake-up time) of the user in advance and the sleep time pattern and the excretion time information may be compared, so that the apparatus can judge whether it is the before-bedtime urine or the overnight urine. Alternatively, a sensor which can sense a fixed weight (a normal weight of a person) may be placed under bedclothes of the user. When the sensor detects the weight continuously for more than a certain period of time (half an hour or longer), it is judged that the bedtime has started. When the sensor has not detected the weight for more than a certain period of time (half an hour or longer), it is judged that the user woke up. This result is sent via a wired or wireless connection from an input/output port included in the measurement apparatus to the measurement apparatus. Then, the result and the excretion time information are compared, so that it is judged whether the excretion time is before or after bedtime.

In this case, as a wired connection method, a USB connection or a LAN (registered trademark) connection can be used, for example. As a wireless connection method, infrared communication, Bluetooth (registered trademark), or a wireless LAN can be used.

Moreover, the display unit is not particularly limited as long as it can display the measurement data and the analysis data. The unit including a liquid crystal display may be used, for example.

Furthermore, it is often the case that a urine component whose amount can be detected as a health condition of the user is given a standard amount as a total daily excretion amount. For example, a target value of the salt intake is 10 g or less per day according to Kenko Nippon 21, and is 5 g or less according to WHO. The standard value is not particularly limited, and an average value previously recommended by a medical institution or the like may be used. Alternatively, the user may directly perform the input of a target value. The comparison unit is not particularly limited, and the CPU or the like may execute the comparison in accordance with a stored program, or the standard value may be displayed together with the daily excretion in order for the user to make a judgment. Moreover, the hard disk may store a health advice corresponding to a result of the comparison between the daily urinary excretion and the standard value. Then, the health advice corresponding to the result of the comparison between the daily urinary excretion and the standard value may be extracted, and the health advice may be displayed on the control panel together with the measurement result and the comparison result. As the health advice, various kinds of advice can be considered corresponding to the condition of the user and the subject of measurement. When the subject of measurement is salt, there may be: an advice informing that excessive salt intake can have an adverse effect on the health of the user; and a tip on limitation of salt intake in daily diet. Or, when the excretion amount is less than the standard value or the target value, a compliment message or the like may be displayed.

This measurement notification unit encourages that the urine measurement be performed at the time represented by the excretion time information having a high correlation with to the daily urinary excretion. The notification method is not particularly limited, and notification by a sound through an alarm generation or notification by light, for example, is made at the time represented by the excretion time information having a high correlation with the daily urinary sodium amount, so as to alert and remind the user of the measurement. Alternatively, as a standby display of the display unit, the excretion time information having a high correlation with the daily urinary sodium amount may be displayed. The user may perform the input of this measurement timing, or one of the recommended time zones stored previously in the apparatus may be selected.

It should be noted that, as a method of detecting the concentration of sodium, various well-known methods can be applied. For example, selective electrodes which selectively react with sodium ion can be used, or a flame photometry can be used. According to the flame photometry, urine as a sample is sprayed into the flame and the intensity of light of a specific wavelength emitted from sodium is measured. Alternatively, the concentration of sodium may be detected through chloride ion measurement. For measuring chloride ion, various well-known methods can be applied, such as a coulometric titration method and an ion selective electrode method.

The amount of urine may be detected by a well-known electronic measurement unit, pressure sensor, or water level sensor. Or, a urine amount detection sensor may be included in the urine collection container. Alternatively, the user may detect the amount of urine by collecting the urine in a graduated container when urinating or by measuring the amount of urine using an electronic measurement apparatus, and then may perform the input directly from the control panel.

As a liquid measurement apparatus, there are known methods including: a method of measuring a liquid level using light and a sound wave; a method whereby a floater is put into the liquid and the position of the floater is measured; a method of measuring the fluid pressure at the bottom of a tank; a method whereby two electrodes are immersed in the liquid and a change in the electric capacitance is used; a method whereby an electrode is immersed in the liquid and the electrical conductivity of the liquid is measured; and a method whereby a resistor is immersed in the liquid and the resistance of the resistor is measured. Also, a weight and a specific gravity of the urine may be measured so that the amount of urine can be measured from the weight and the specific gravity.

In the above embodiments, when the measurement start button 204 is pressed in step S301 in FIG. 4, the current time of measurement is obtained from the timer unit 216 and then the user performs the input of the excretion time information by pressing the excretion time information button 205. However, the user may manually perform only the input of the current time of measurement, or only the input of the excretion time information.

It should be noted that all the function blocks shown in the block diagrams (FIGS. 3, 8, 10, and 13) and the control unit 2 shown in FIGS. 2 and 12 are realized as an LSI which is typically an integrated circuit device. These may be individually integrated into one chip or may be integrated into one chip including some or all of them. For example, the function blocks except for the memory may be integrated into one chip.

Although referred to as the LSI here, it may be referred to as an IC, a system LSI, a super LSI, or an ultra LSI depending on the scale of integration.

A method for circuit integration is not limited to application of an LSI. It may be realized using a dedicated circuit or a general purpose processor. After an LSI is manufactured, an FPGA (Field Programmable Gate Array) which is programmable or a reconfigurable processor for which the connections and settings of circuit cells inside the LSI are reconfigurable may be used.

Moreover, when a circuit integration technology that replaces LSIs comes along owing to advances of the semiconductor technology or to a separate derivative technology, the function blocks should be understandably integrated using that technology. There can be a possibility of adaptation of biotechnology, for example.

Furthermore, among all the function blocks, only the unit storing the data which can be encoded or decoded may not be integrated into the single chip and separately configured.

## Industrial Applicability

The daily urinary excretion measurement apparatus and the daily urinary excretion measurement method of the present invention are useful as an apparatus and a method whereby a daily excretion of a urine component is measured highly accurately and easily and a total daily excretion of the urine component is estimated. The present invention can also be applied to healthcare, such as salt intake management, carried out using urine samples.

## Claims

1. A daily urinary excretion measurement method for measuring a total amount of a urine component excreted in daily urine, comprising:

    collecting a plurality of spot urine samples (each of which is collected per urination) out of spot urine samples excreted within a predetermined period of time, and calculating an amount of the urine component for each spot urine sample using the collected plurality of spot urine samples, said collecting and said calculating being performed by a urine component calculation unit; and
    holding a correlation between the amounts of the urine component included in the plurality of spot urine samples excreted within the predetermined period of time and a daily urinary excretion which is the total amount of the urine component included in the daily urine, and calculating the daily urinary excretion on the basis of the correlation and the amounts of the urine component included in the plurality of spot urine samples calculated by said urine component calculation unit, said holding and said calculating of the daily urinary excretion being performed by a daily urinary excretion calculation unit.

2. The daily urinary excretion measurement method according to Claim 1,
wherein, in said calculating of the daily urinary excretion, the daily urinary excretion calculation unit calculates the daily urinary excretion of the urine component using the amounts of the urine component included in two of the spot urine samples collected within the predetermined period of time.

3. The daily urinary excretion measurement method according to Claim 2,
wherein, in said calculating of the amount of the urine component, the urine component calculation unit calculates the amount of the urine component for each of the plurality of spot urine samples using the plurality of spot urine samples excreted within twenty-four hours.

4. The daily urinary excretion measurement method according to Claim 3,
wherein, in said calculating of the amount of the urine component, the urine component calculation unit calculates the amount of the urine component for each of the plurality of spot urine samples using a component concentration of the spot urine sample measured by a component sensor and a urine amount measured by a urine amount sensor.

5. The daily urinary excretion measurement method according to Claim 4,
wherein, in said calculating of the amount of the urine component, the urine component calculation unit calculates the amount of the urine component for each of the plurality of spot urine samples of before-bedtime urine and overnight urine, the before-bedtime urine being excreted and collected for the last time before bedtime and the overnight urine being excreted and collected for the first time after wake-up following a sleep, and
in said calculating of the daily urinary excretion, the daily urinary excretion calculation unit calculates the daily urinary excretion of the urine component using the amount of the before-bedtime urine component calculated by the urine component calculation unit and the amount of the overnight urine component calculated by the urine component calculation unit, on the basis of a correlation between: the amount of the before-bedtime urine component and the amount of the overnight urine component; and the total amount of the urine component included in the daily urine.

6. The daily urinary excretion measurement method according to Claim 5, further comprising:

    receiving an input of excretion time information regarding an excretion time of the spot urine sample, said receiving being performed by an input unit;
    accumulating the amount of the urine component calculated by the urine component calculation unit for each of the spot urine samples in association with the excretion time information of the spot urine sample received by the input unit; and
    performing an 8-hour correction on the amount of the overnight urine component by converting a period of time taken from an excretion time of the before-bedtime urine to an excretion time of the overnight urine into 8 hours, on the basis of the pieces of the excretion time information accumulated respectively in association with the amount of before-bedtime urine component and the amount of the overnight urine component,

wherein, in said calculating of the daily urinary excretion, the daily urinary excretion calculation unit calculates the daily urinary excretion of the urine component using: the amount of the before-bedtime urine component calculated by the urine component calculation unit; and the amount of the overnight urine component calculated by the urine component calculation unit and corrected by the daily urinary excretion calculation unit in said performing of the 8-hour correction, on the basis of a correlation between: a sum of the before-bedtime urine component amount and the 8-hour corrected overnight urine component amount; and the total amount of the urine component included in the daily urine.

**7.** The daily urinary excretion measurement method according to Claim 1,
wherein, in said calculating of the amount of the urine component, the urine component calculation unit calculates a ratio of the urine component to creatinine in the spot urine sample as the amount of the urine component included in the spot urine sample, from a component concentration of the spot urine sample measured by a component sensor and a creatinine concentration of the spot urine sample measured by a creatinine sensor, and
in said calculating of the daily urinary excretion, the daily urinary excretion calculation unit calculates the daily urinary excretion of the urine component, using: the creatinine ratio which is calculated as the amount of the urine component by the urine component calculation unit; and an amount of daily creatinine excretion held in advance by the urine component calculation unit.

**8.** The daily urinary excretion measurement method according to Claim 7,
wherein, in said calculating of the amount of the daily urinary excretion, the daily urinary excretion calculation unit calculates the daily urinary excretion using the amounts of the urine component of at least three but not exceeding fourteen spot urine samples calculated by the urine component calculation unit within the predetermined period of time.

**9.** The daily urinary excretion measurement method according to Claim 8,
wherein, in said calculating of the amount of the urine component, the urine component calculation unit calculates the amounts of the urine component of at least three but not exceeding fourteen spot urine samples collected out of the spot urine samples excreted in seven days.

**10.** The daily urinary excretion measurement method according to Claim 9,
wherein, in said calculating of the amount of the urine component, the urine component calculation unit calculates the amounts of the urine component of the spot urine samples including at least two pairs of the before-bedtime urine sample and the overnight urine sample out of the collected spot urine samples, each of the pairs sandwiching a same sleep.

**11.** The daily urinary excretion measurement method according to Claim 9,
wherein, in said calculating of the amount of the urine component, the urine component calculation unit calculates the amounts of the urine component of the collected spot urine samples, all of which are the overnight urine samples.

**12.** The daily urinary excretion measurement method according to Claim 9,
wherein, in said calculating of the amount of the urine component, the urine component calculation unit calculates the amounts of the urine component of the collected spot urine samples, all of which are the before-bedtime urine samples.

**13.** A daily urinary excretion measurement apparatus which measures a total amount of a urine component excreted in daily urine, comprising:

a urine component calculation unit configured to collect a plurality of spot urine samples (each of which is collected per urination) out of spot urine samples excreted within a predetermined period of time, and to calculate an amount of a urine component for each spot urine sample using the collected plurality of spot urine samples; and
a daily urinary excretion calculation unit configured to hold a correlation between the amounts of the urine component included in the plurality of spot urine samples excreted within the predetermined period of time and a daily urinary excretion which is the total amount of the urine component included in the daily urine, and to calculate the daily urinary excretion on the basis of the correlation and the amounts of the urine component included in the plurality of spot urine samples calculated by said urine component calculation unit.

**14.** The daily urinary excretion measurement apparatus according to Claim 13,
wherein said urine component calculation unit includes:

a component sensor configured to measure a component concentration of the spot urine sample; and
a urine amount sensor configured to measure a urine amount of the spot urine sample, and
said urine component calculation unit is configured to calculate the amount of the urine component included in the spot urine sample from the component concentration measured by said component sensor and the urine amount measured by said urine amount sensor.

**15.** The daily urinary excretion measurement apparatus according to Claim 13,
wherein said urine component calculation unit includes:

a component sensor configured to measure a component concentration of the spot urine sample; and
a creatinine sensor configured to measure a creatinine concentration of the spot urine sample, and
said urine component calculation unit is configured to calculate a ratio of the urine component to creatinine in the spot urine sample as the amount of the urine component included in the spot urine sample, from the component concentration measured by said component sensor and the creatinine concentration measured by said creatinine sensor.

**16.** The daily urinary excretion measurement apparatus according to Claim 13, further comprising
a display unit configured to display one of: each amount of the urine component in the plurality of spot urine samples calculated by said urine component calculation unit; and the daily urinary excretion calculated by said daily urinary excretion calculation unit.

**17.** The daily urinary excretion measurement apparatus according to Claim 13,
wherein said urine component calculation unit is configured to calculate the amount of the urine component for each of the plurality of spot urine samples of before-bedtime urine and overnight urine, the before-bedtime urine being excreted and collected for the last time before bedtime and the overnight urine being excreted and collected for the first time after wake-up following a sleep, and
said daily urinary excretion measurement apparatus further comprising
a display unit configured to display one of: the amount of the before-bedtime urine component calculated by said urine component calculation unit; the amount of the overnight urine component calculated by said urine component calculation unit; and the daily urinary excretion calculated by said daily urinary excretion calculation unit.

**18.** The daily urinary excretion measurement apparatus according to Claim 13, further comprising
a display unit configured to display the daily urinary excretion calculated by said daily urinary excretion calculation unit only after said urine component calculation unit calculates the amount of the urine component for each of the plurality of spot urine samples collected within the predetermined period of time.

**19.** The daily urinary excretion measurement apparatus according to Claim 13, further comprising:

a standard amount reception unit configured to receive an input of a standard amount of the total amount of the urine component excreted in the daily urine;
a comparison unit configured to compare the daily urinary excretion calculated by said daily urinary excretion calculation unit and the standard amount received by said standard amount reception unit, and to judge whether or not the calculated daily urinary excretion exceeds the standard amount; and
a display unit configured to display an advice when said comparison unit judges that the daily urinary excretion exceeds the standard amount.

**20.** The daily urinary excretion measurement apparatus according to Claim 13, further comprising
a measurement notification unit configured to notify a user of a timing at which the amount of the urine component is to be calculated by said urine component calculation unit.

**21.** The daily urinary excretion measurement apparatus according to Claim 13, further comprising:

a urine component accumulation unit configured to accumulate the amounts of the before-bedtime urine component and the amounts of the overnight urine component of at least three days together with the respective corresponding daily urinary excretions; and
a correlation calculation unit configured to calculate, from: a sum of the amount of the before-bedtime urine component and the amount of the overnight urine component; and the corresponding daily urinary excretion accumulated in said urine component accumulation unit, a correlation between: the sum of the amount of the before-bedtime urine component and the amount of the overnight urine component; and the corresponding daily urinary excretion according to a linear approximation.

**22.** A program used for measuring a total amount of a urine component excreted in daily urine, said program causing a computer to execute:

collecting a plurality of spot urine samples (each of which is collected per urination) out of spot urine samples excreted within a predetermined period of time, and calculating an amount of the urine component for each spot urine sample using the collected plurality of spot urine samples; and
holding a correlation between the amounts of the urine component included in the plurality of spot urine samples excreted within the predetermined period of time and a daily urinary excretion which is the total amount of the urine component included in the daily urine, and calculating the daily urinary excretion on the basis of the correlation and the amounts of the urine component included in the plurality of spot urine samples calculated by said urine component calculation unit.

FIG. 1

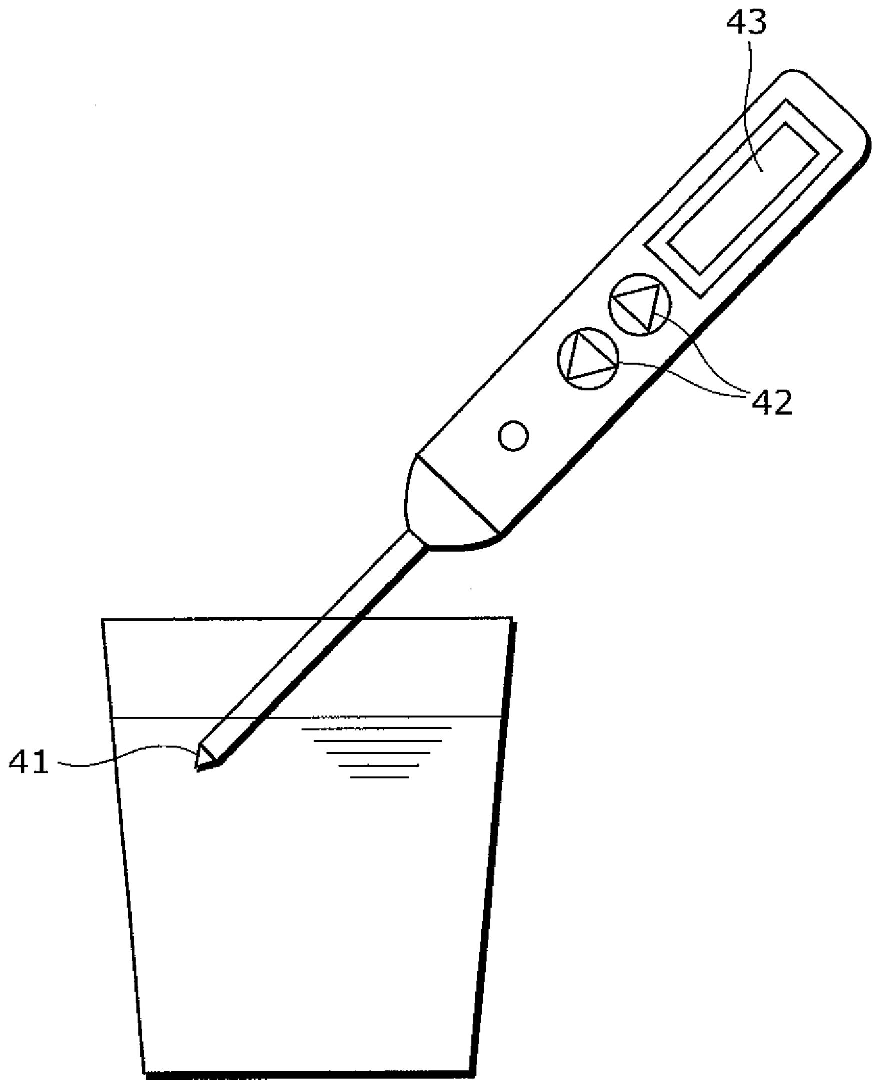

FIG. 2

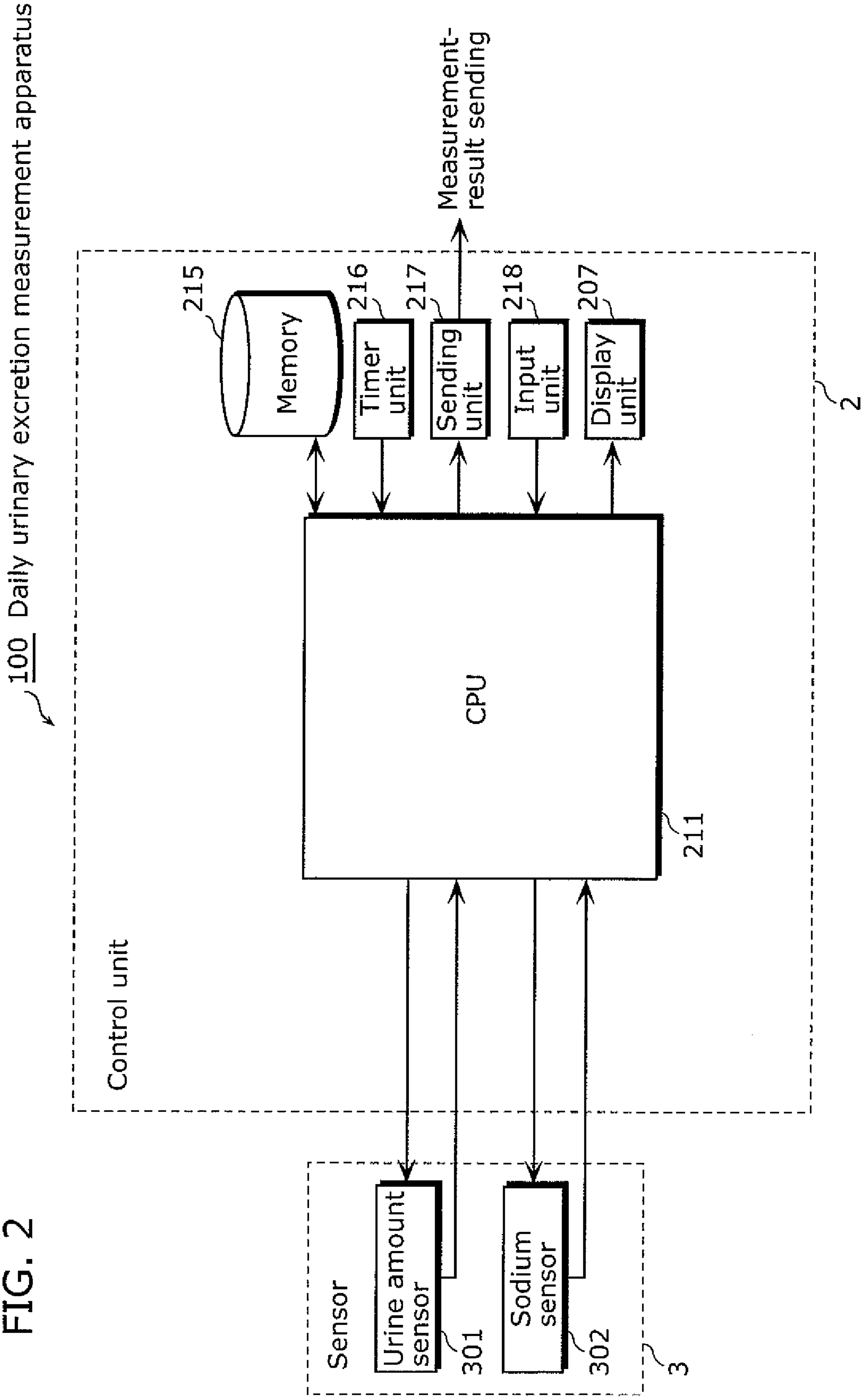
100 Daily urinary excretion measurement apparatus
Control unit
2
CPU
211
Memory
215
Timer unit
216
Sending unit
217
Input unit
218
Display unit
207
Measurement-result sending
Sensor
3
Urine amount sensor
301
Sodium sensor
302

FIG. 3

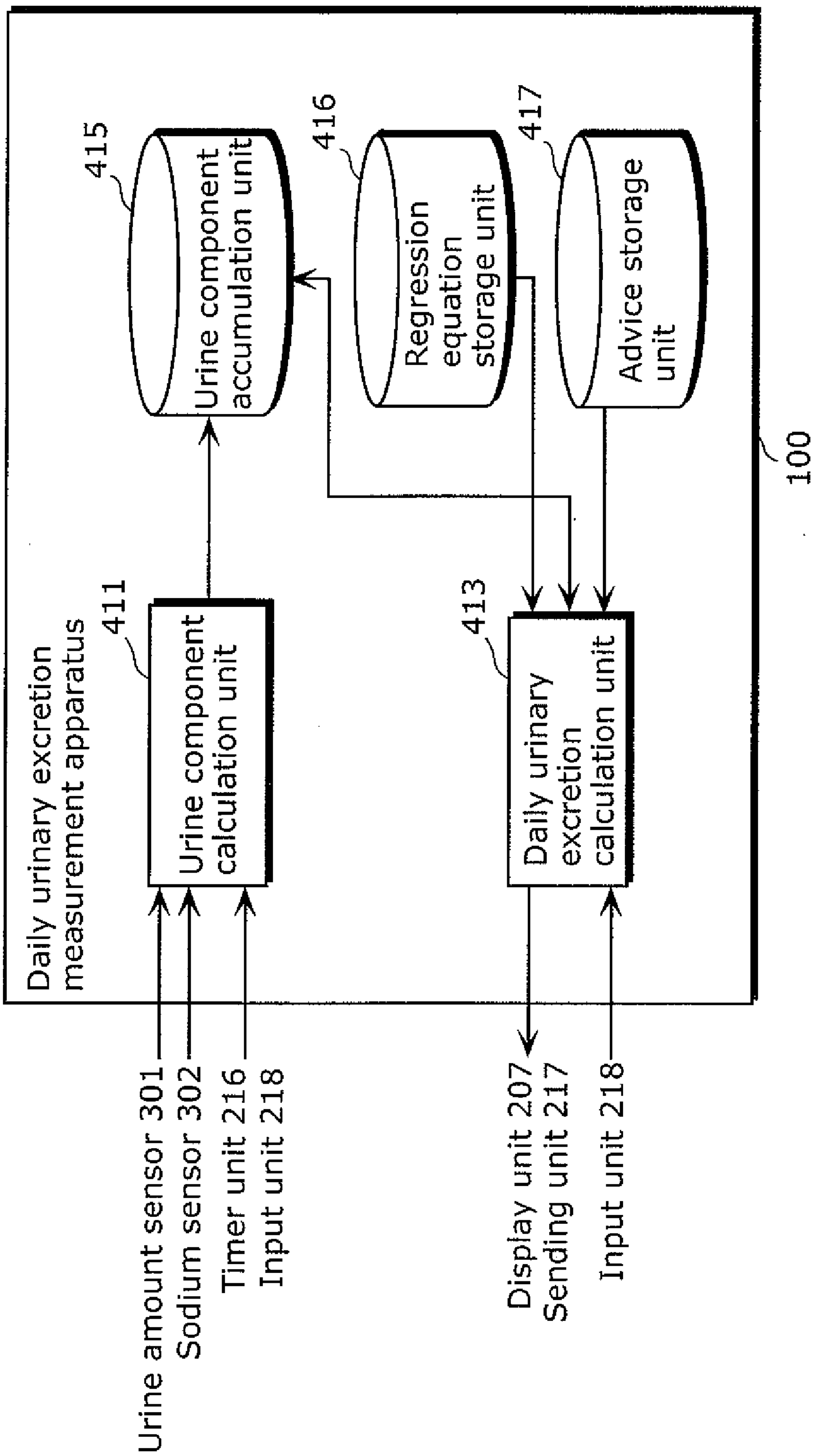
Daily urinary excretion measurement apparatus
100
411
Urine component calculation unit
413
Daily urinary excretion calculation unit
415
Urine component accumulation unit
416
Regression equation storage unit
417
Advice storage unit
Urine amount sensor 301
Sodium sensor 302
Timer unit 216
Input unit 218
Display unit 207
Sending unit 217
Input unit 218

FIG. 4

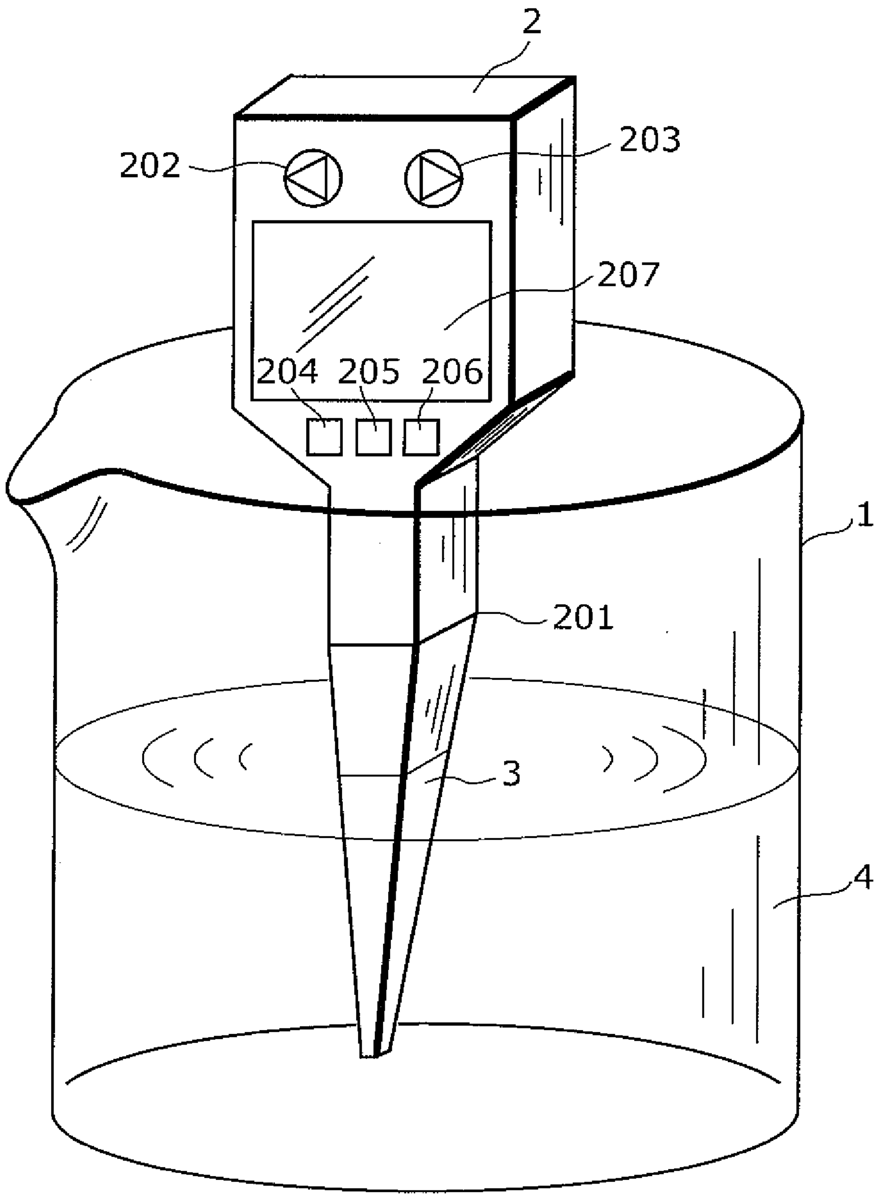

FIG. 5

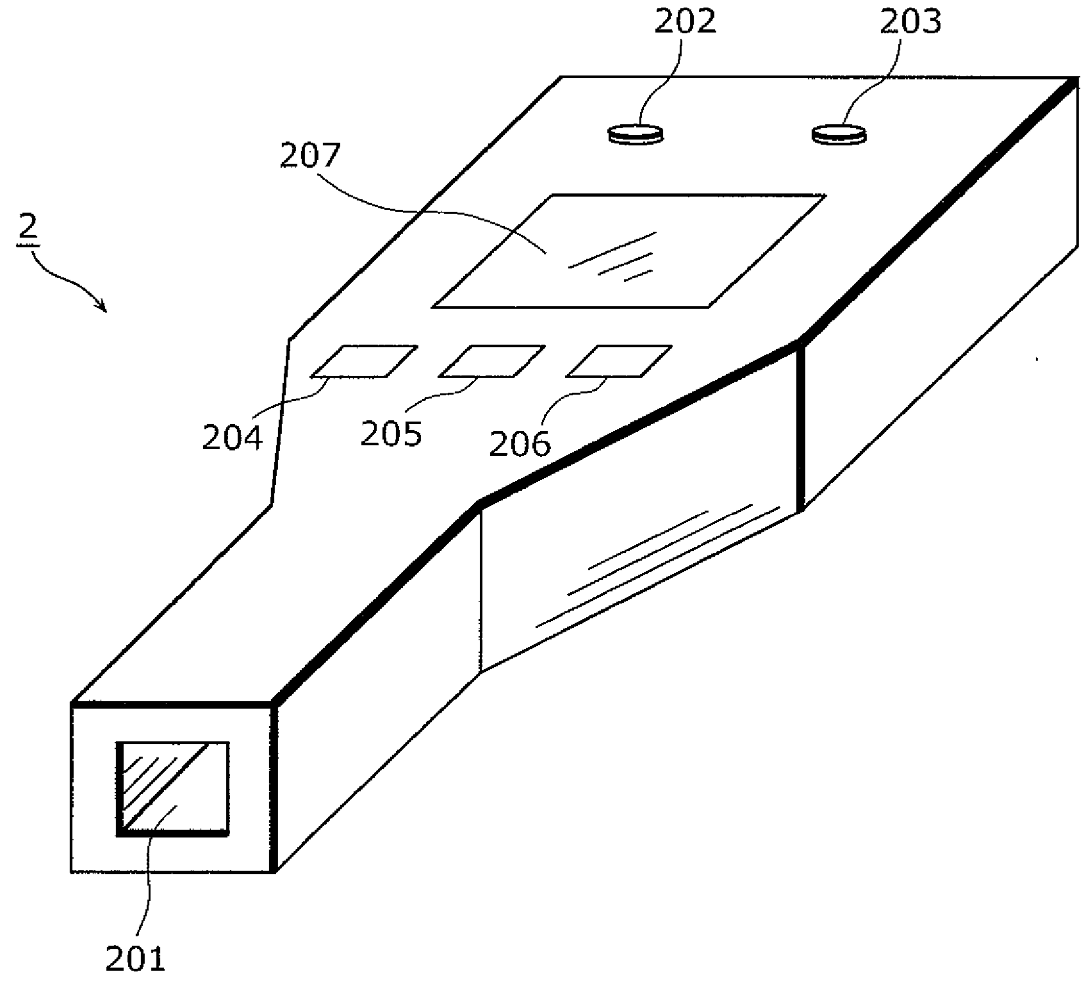

FIG. 6

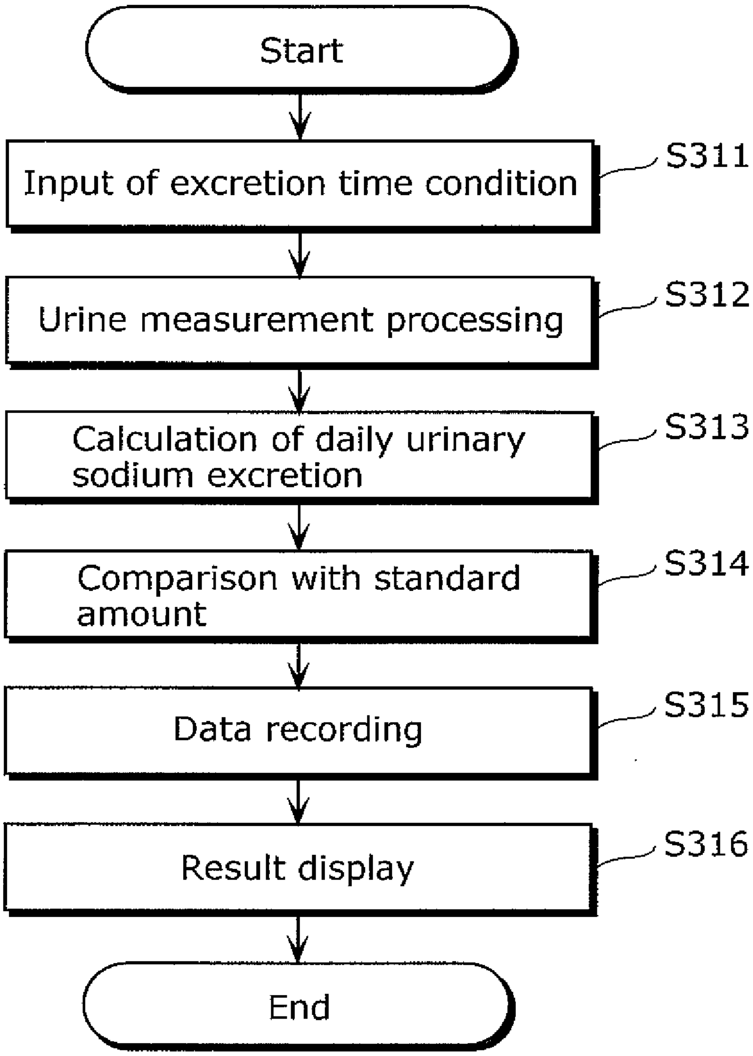
Start
Input of excretion time condition
S311
Urine measurement processing
S312
Calculation of daily urinary sodium excretion
S313
Comparison with standard amount
S314
Data recording
S315
Result display
S316
End

FIG. 7

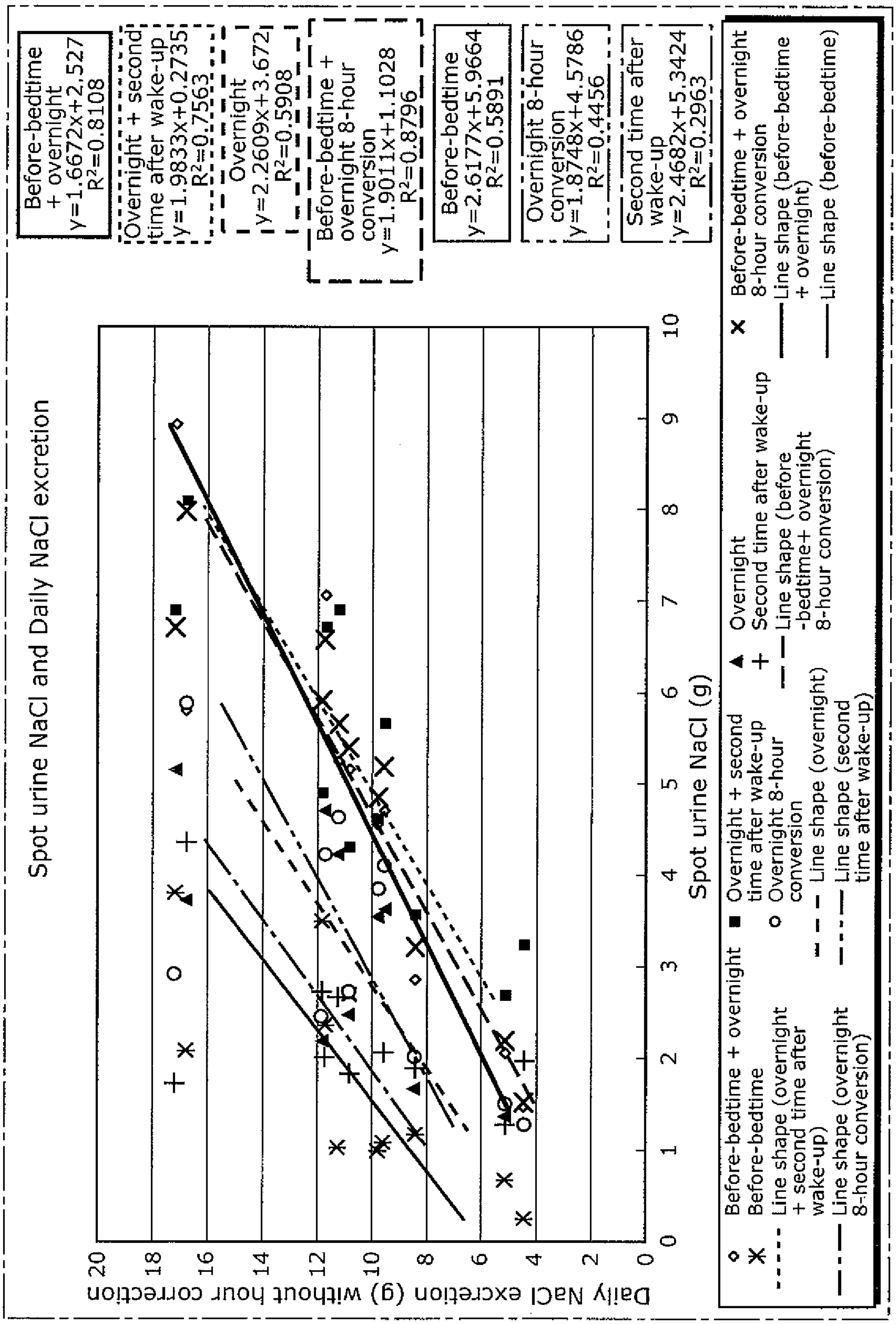
Spot urine NaCl and Daily NaCl excretion
Daily NaCl excretion (g) without hour correction
Spot urine NaCl (g)
0
2
4
6
8
10
12
14
16
18
20
0
1
2
3
4
5
6
7
8
9
10
Before-bedtime + overnight
y=1.6672x+2.527
R²=0.8108
Overnight + second time after wake-up
y=1.9833x+0.2735
R²=0.7563
Overnight
y=2.2609x+3.672
R²=0.5908
Before-bedtime + overnight 8-hour conversion
y=1.9011x+1.1028
R²=0.8796
Before-bedtime
y=2.6177x+5.9664
R²=0.5891
Overnight 8-hour conversion
y=1.8748x+4.5786
R²=0.4456
Second time after wake-up
y=2.4682x+5.3424
R²=0.2963
Before-bedtime + overnight
Before-bedtime
Overnight + second time after wake-up
Overnight 8-hour conversion
Overnight
Second time after wake-up
Before-bedtime + overnight 8-hour conversion
Line shape (overnight + second time after wake-up)
Line shape (overnight 8-hour conversion)
Line shape (overnight)
Line shape (second time after wake-up)
Line shape (before -bedtime+ overnight 8-hour conversion)
Line shape (before-bedtime + overnight)
Line shape (before-bedtime)

FIG. 8

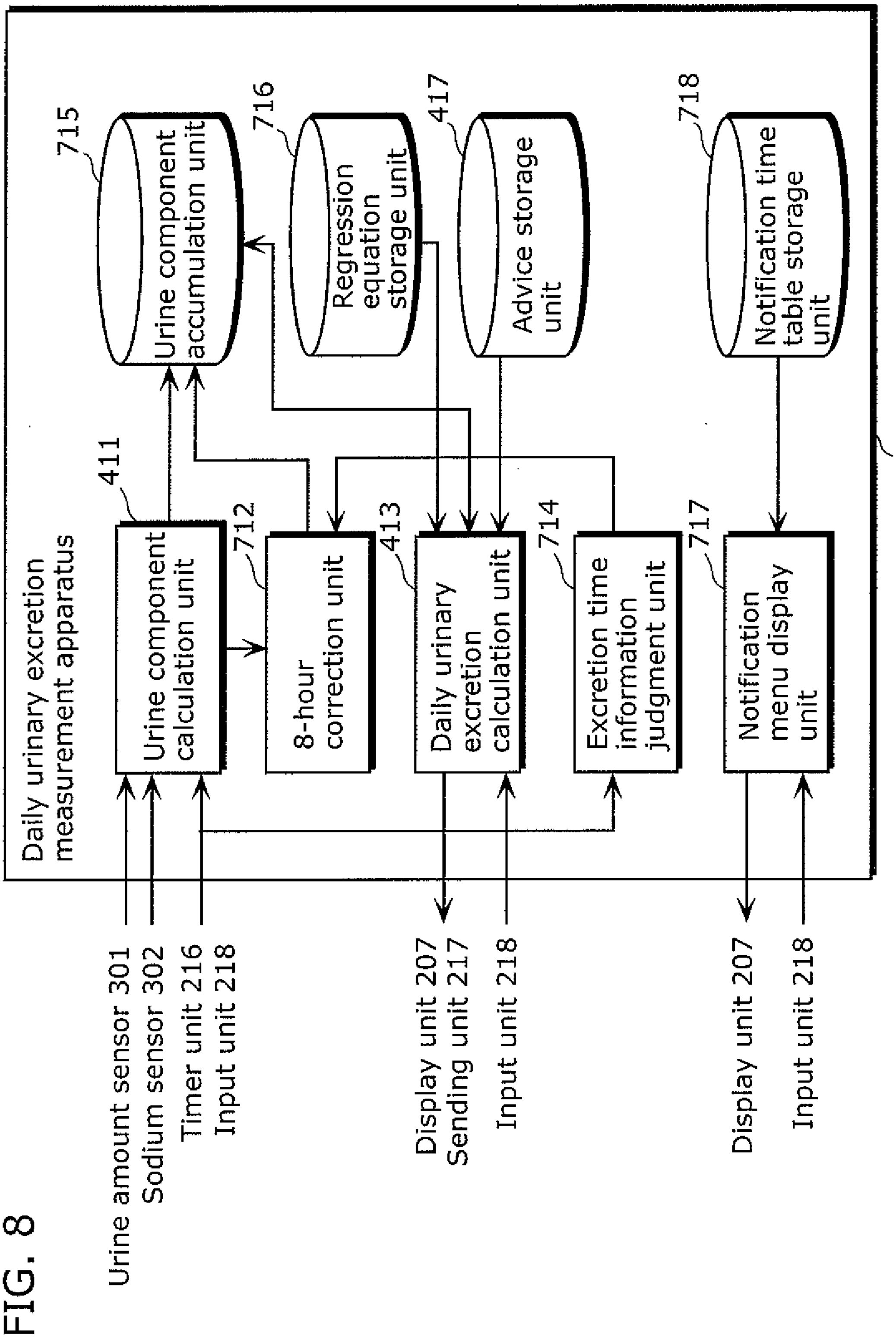
Daily urinary excretion measurement apparatus
200
Urine component calculation unit
411
8-hour correction unit
712
Daily urinary excretion calculation unit
413
Excretion time information judgment unit
714
Notification menu display unit
717
Urine component accumulation unit
715
Regression equation storage unit
716
Advice storage unit
417
Notification time table storage unit
718
Urine amount sensor 301
Sodium sensor 302
Timer unit 216
Input unit 218
Display unit 207
Sending unit 217
Input unit 218
Display unit 207
Input unit 218

FIG. 9

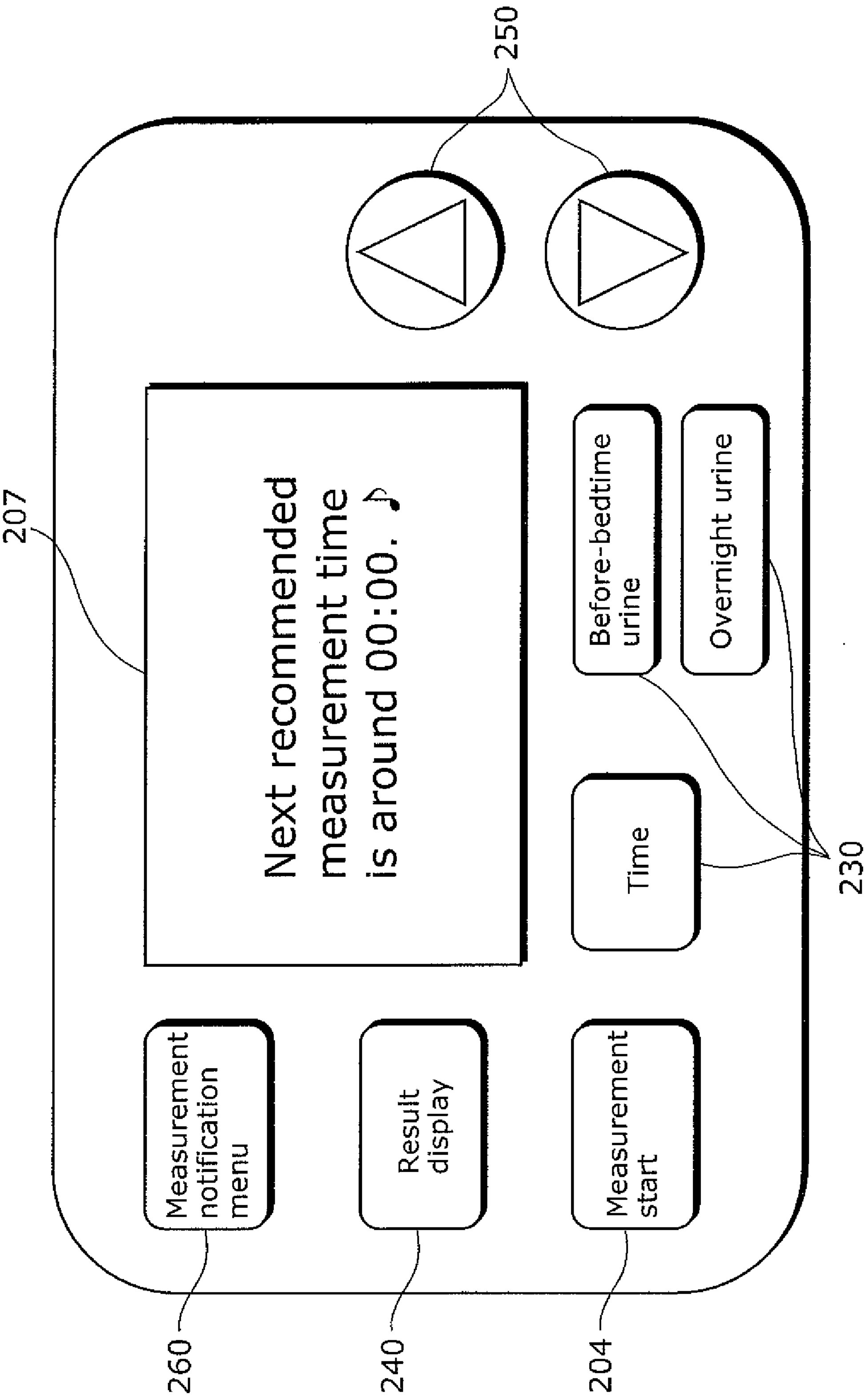
250
207
Next recommend measurement time is around 00:00. ♪
Measurement notification menu
Result display
Measurement start
Time
Before-bedtime urine
Overnight urine
230
260
240
204

FIG. 10

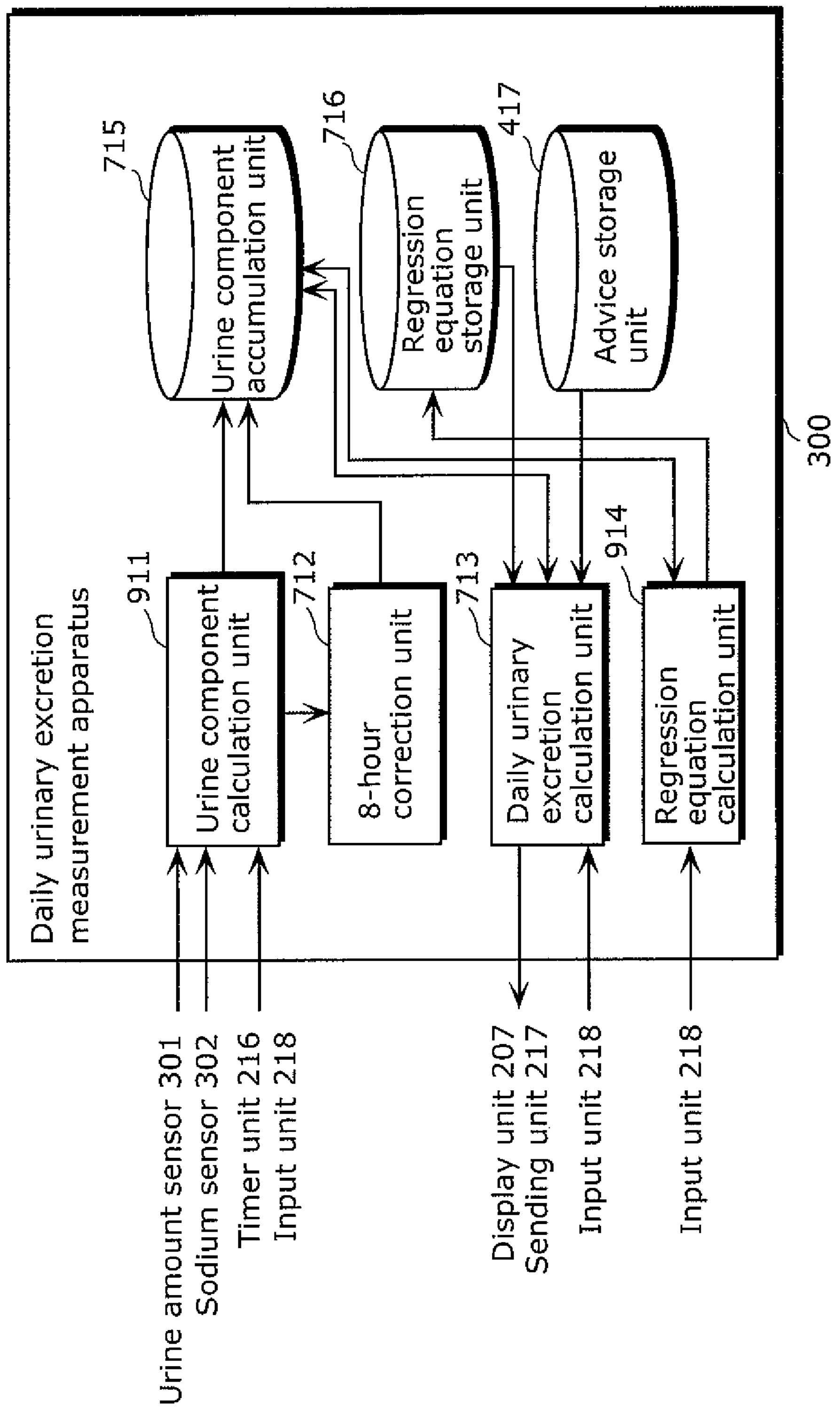
Daily urinary excretion measurement apparatus
911
Urine component calculation unit
712
8-hour correction unit
713
Daily urinary excretion calculation unit
914
Regression equation calculation unit
715
Urine component accumulation unit
716
Regression equation storage unit
417
Advice storage unit
300
Urine amount sensor 301
Sodium sensor 302
Timer unit 216
Input unit 218
Display unit 207
Sending unit 217
Input unit 218
Input unit 218

FIG. 11

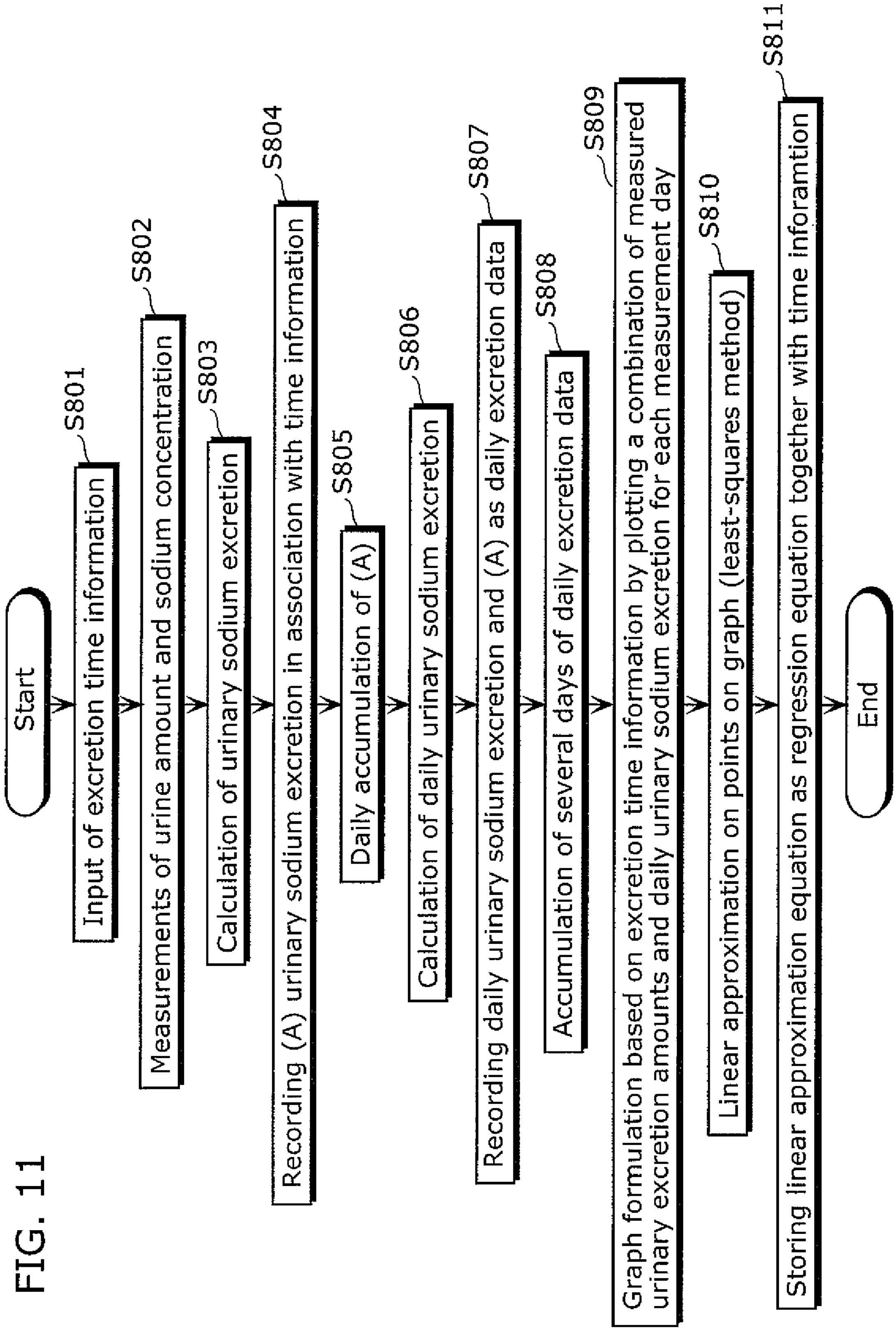
Start
S801
Input of excretion time information
S802
Measurements of urine amount and sodium concentration
S803
Calculation of urinary sodium excretion
S804
Recording (A) urinary sodium excretion in association with time information
S805
Daily accumulation of (A)
S806
Calculation of daily urinary sodium excretion
S807
Recording daily urinary sodium excretion and (A) as daily excretion data
S808
Accumulation of several days of daily excretion data
S809
Graph formulation based on excretion time information by plotting a combination of measured urinary excretion amounts and daily urinary sodium excretion for each measurement day
S810
Linear approximation on points on graph (least-squares method)
S811
Storing linear approximation equation as regression equation together with time inforamtion
End

FIG. 12

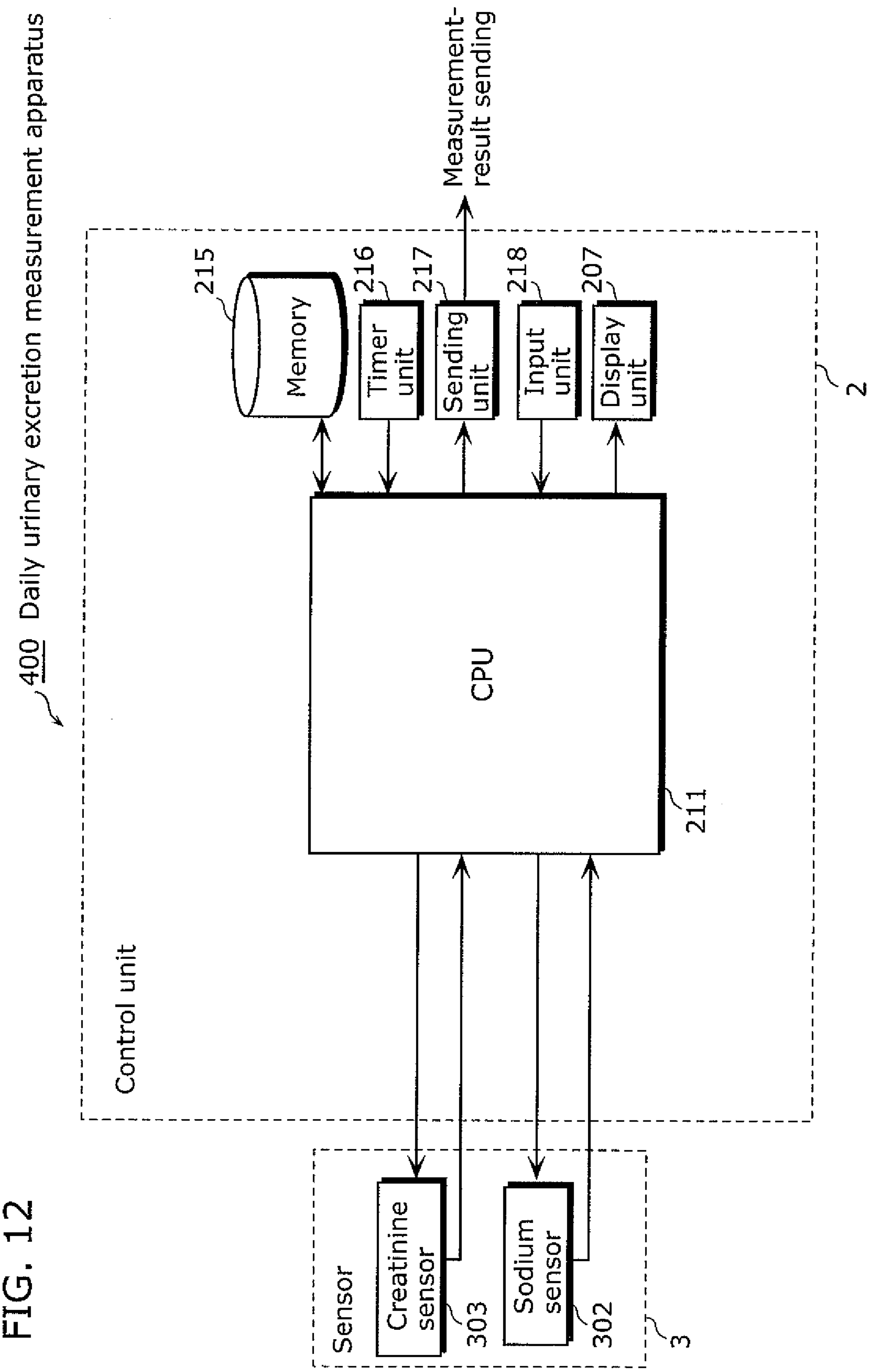
400 Daily urinary excretion measurement apparatus
Control unit
2
Memory
215
Timer unit
216
Sending unit
217
Measurement-result sending
Input unit
218
Display unit
207
CPU
211
Sensor
3
Creatinine sensor
303
Sodium sensor
302

FIG. 13

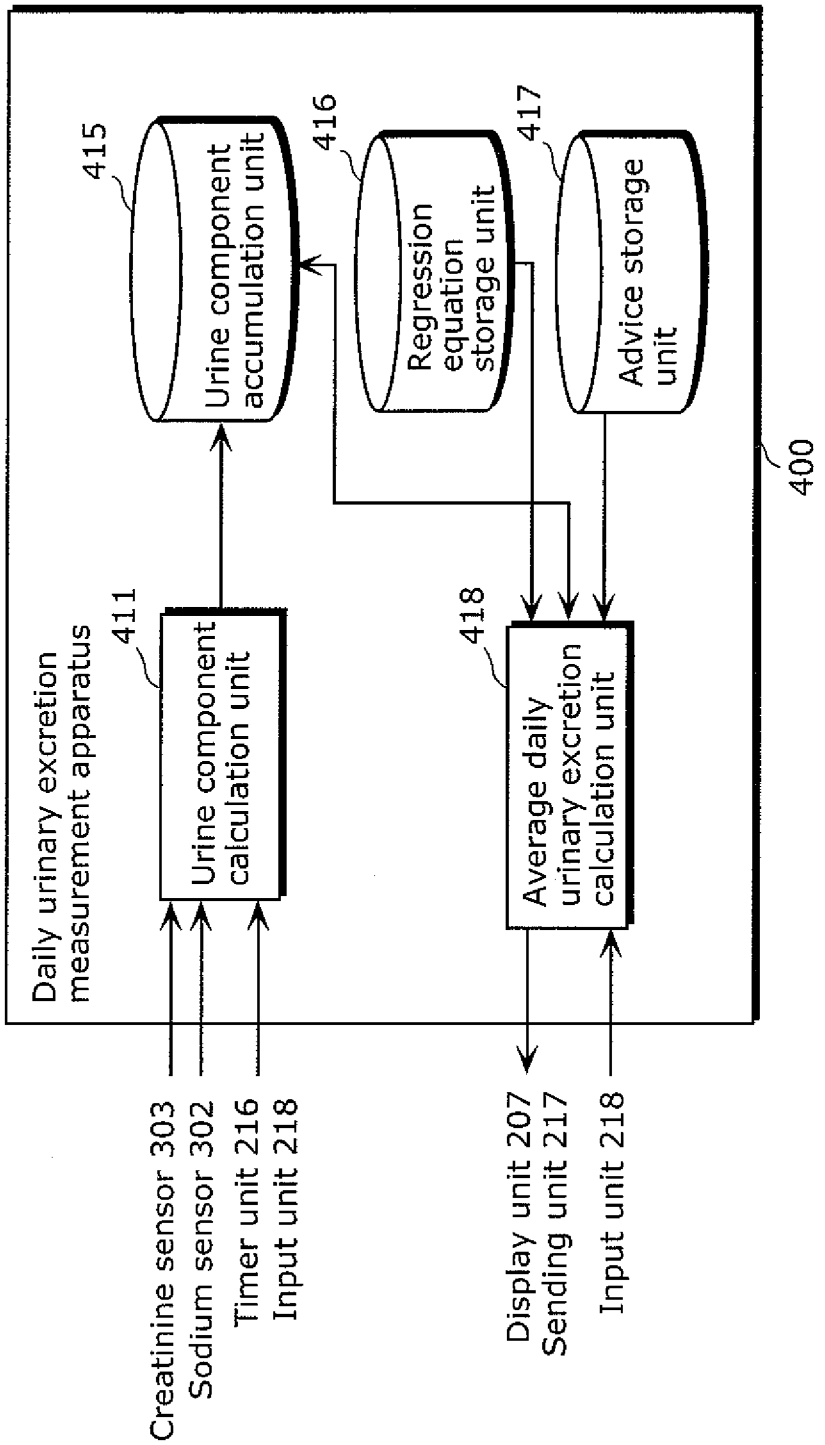
Daily urinary excretion measurement apparatus
400
411
Urine component calculation unit
415
Urine component accumulation unit
416
Regression equation storage unit
417
Advice storage unit
418
Average daily urinary excretion calculation unit
Creatinine sensor 303
Sodium sensor 302
Timer unit 216
Input unit 218
Display unit 207
Sending unit 217
Input unit 218

FIG. 14

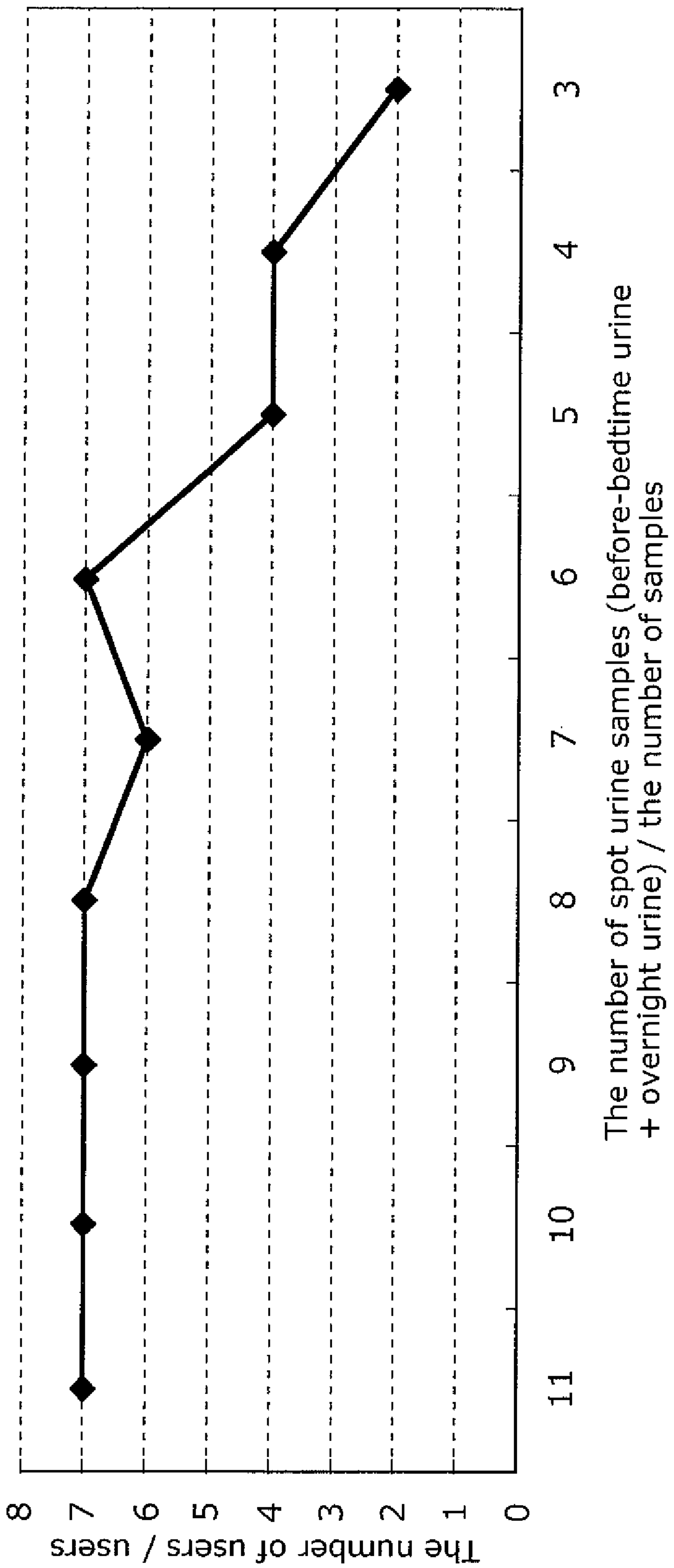
The number of users / users
0
1
2
3
4
5
6
7
8
11
10
9
8
7
6
5
4
3
The number of spot urine samples (before-bedtime urine
+ overnight urine) / the number of samples

FIG. 15

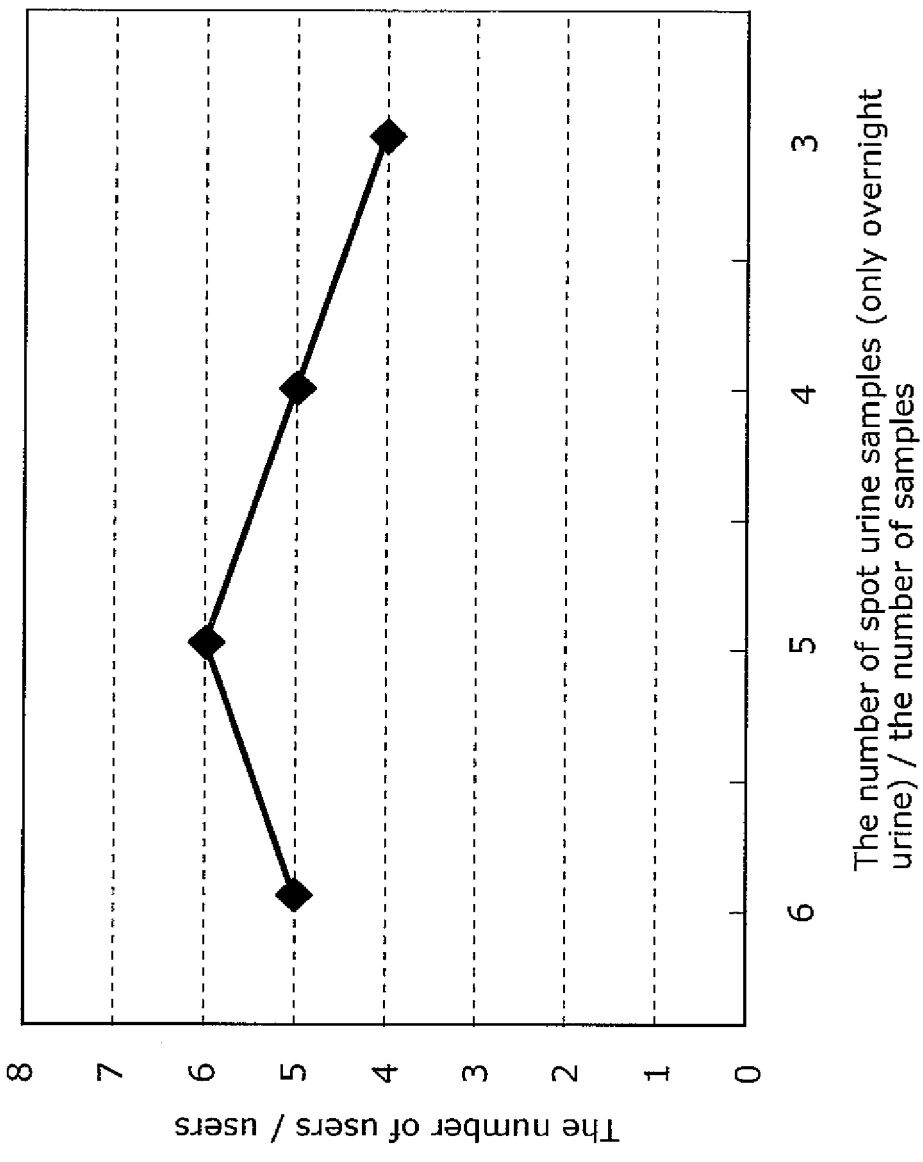
The number of users / users
0
1
2
3
4
5
6
7
8
6
5
4
3
The number of spot urine samples (only overnight urine) / the number of samples

FIG. 16

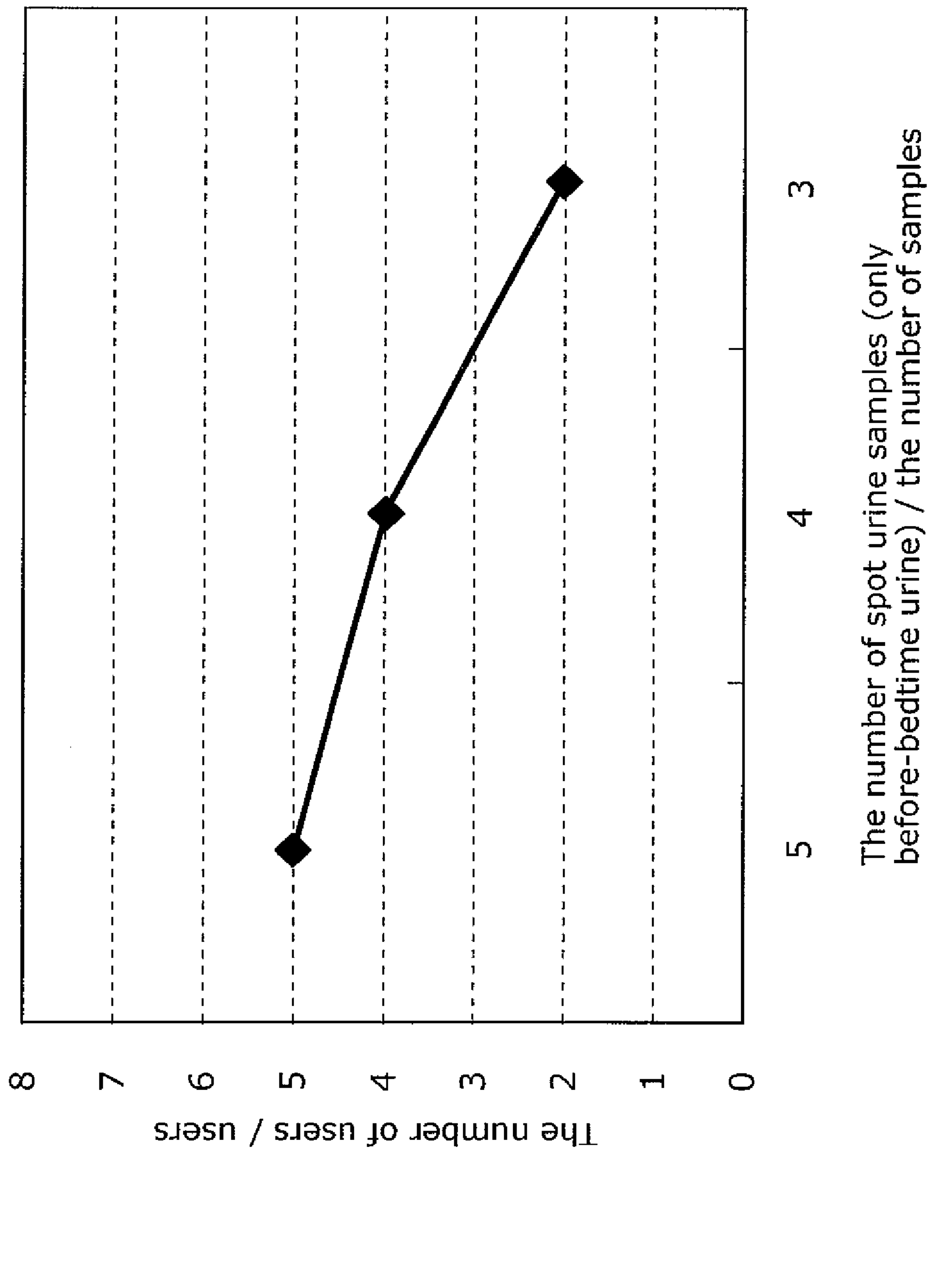
The number of users / users
0
1
2
3
4
5
6
7
8
5
4
3
The number of spot urine samples (only before-bedtime urine) / the number of samples

**INTERNATIONAL SEARCH REPORT**

International application No.
PCT/JP2007/058883

A. CLASSIFICATION OF SUBJECT MATTER
*G01N33/493*(2006.01)i

According to International Patent Classification (IPC) or to both national classification and IPC

B. FIELDS SEARCHED

Minimum documentation searched (classification system followed by classification symbols)
G01N33/493

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X /Y | JP 2005-024267 A (Toto Ltd.), 27 January, 2005 (27.01.05), Par. Nos. [0008], [0009]; Fig. 8 (Family: none) | 1-5,13-14,22 /6-12,15-21 |
| Y | JP 2002-214186 A (NEC Corp.), 31 July, 2002 (31.07.02), Par. Nos. [0009], [0041], [0063] (Family: none) | 6,19 |
| Y | JP 2006-029819 A (Toto Ltd.), 02 February, 2006 (02.02.06), Par. Nos. [0008], [0044] to [0045], [0051], [0054]; Fig. 13 (Family: none) | 6,19 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 22 May, 2007 (22.05.07) | 29 May, 2007 (29.05.07) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2007/058883

C (Continuation). DOCUMENTS CONSIDERED TO BE RELEVANT

| Category* | Citation of document, with indication, where appropriate, of the relevant passages | Relevant to claim No. |
|---|---|---|
| Y | Kotaro YAMASUE et al., "Katei deno Enbun, Kalium Sesshuryo Sokuteiho no Kento", Nippon Junkankibyo Yobo Gakkaishi, 30 October, 2004 (30.10.04), Vol.39, No.3, pages 157 to 163, abstract, page 157, lower right column, lines 9 to 12, page 158, right column, line 6, page 161, right column, lines 40 to 48 | 6-12,15-18, 21 |
| Y | JP 2005-291873 A (Matsushita Electric Industrial Co., Ltd.), 20 October, 2005 (20.10.05), Par. Nos. [0003] to [0005] (Family: none) | 20 |
| A | JP 2001-264321 A (Matsushita Electric Industrial Co., Ltd.), 26 September, 2001 (26.09.01), (Family: none) | 1-22 |
| A | JP 2002-267662 A (Sekisui Chemical Co., Ltd.), 18 September, 2002 (18.09.02), (Family: none) | 1-22 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002267662 A
- JP 54151095 A
- JP 2001512692 A


### Non-patent literature cited in the description


- **KOTARO YAMASUE et al.** Methods for self-monitoring of daily salt and potassium intake at home. *JJCDP,* 2004, vol. 39 (3), 157-163
- **H. UESHIMA et al.** A simple method to estimate populational 24-h urinary sodium and potassium excretion using a casual urine specimen. *Journal of Human Hypertension,* 2002, vol. 16, 97-103